(19) Europäisches Patentamt
European Patent Office
Office européen des brevets

(11) EP 3 558 241 B1

(12) **EUROPEAN PATENT SPECIFICATION**

(45) Date of publication and mention of the grant of the patent:
**07.07.2021 Bulletin 2021/27**

(21) Application number: **17829185.2**

(22) Date of filing: **19.12.2017**

(51) Int Cl.:
***A61K 8/41*** *(2006.01)* ***A61K 8/43*** *(2006.01)*
***A61K 8/72*** *(2006.01)* ***A61K 8/19*** *(2006.01)*
***A61Q 5/08*** *(2006.01)* ***A61Q 5/10*** *(2006.01)*
***A61K 8/34*** *(2006.01)* ***A61K 8/84*** *(2006.01)*

(86) International application number:
**PCT/EP2017/083631**

(87) International publication number:
**WO 2018/115002 (28.06.2018 Gazette 2018/26)**

(54) **HAIR COMPOSITION COMPRISING A GUANIDINE SALT, AN ALKANOLAMINE AND AMMONIUM HYDROXIDE AND CATIONIC AND/OR AMPHOTERIC POLYMERS**

HAARZUSAMMENSETZUNG MIT EIN GUANIDINSALZ, EINEM ALKANOLAMIN- UND AMMONIUMHYDROXID UND KATIONISCHEN UND/ODER AMPHOTEREN POLYMEREN

COMPOSITION CAPILLAIRE COMPRENANT UN SEL DE GUANIDINE, UNE ALCANOLAMINE ET DE L’HYDROXYDE D’AMMONIUM ET DES POLYMÈRES CATIONIQUES ET/OU AMPHOTÈRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2016 FR 1663023**

(43) Date of publication of application:
**30.10.2019 Bulletin 2019/44**

(73) Proprietor: **L’Oreal**
**75008 Paris (FR)**

(72) Inventors:
- **MEGUENI, Amine**
**93400 Saint-Ouen (FR)**
- **HERCOUET, Leila**
**93400 Saint-Ouen (FR)**

(74) Representative: **L’Oreal**
**Service D.I.P.I.**
**9, rue Pierre Dreyfus**
**92110 Clichy (FR)**

(56) References cited:
**EP-A1- 0 890 355** **EP-A2- 1 106 166**
**WO-A1-2015/022360** **WO-A1-2015/122029**
**FR-A1- 2 841 778** **FR-A1- 2 925 305**
**FR-A1- 3 015 240** **FR-A1- 3 015 241**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 558 241 B1

## Description

The present invention relates to a composition in particular for dyeing and/or lightening keratin fibres, in particular human keratin fibres such as the hair, comprising a combination of three alkaline agents chosen from guanidine salts, alkanolamines and ammonium hydroxide and at least two different cationic and/or amphoteric polymers.

The invention also relates to a dyeing and/or lightening process using said composition, and also to a multicompartment device which is suitable for using said dyeing and/or lightening composition.

The present invention relates to the field of lightening keratin fibres and more particularly to the field of dyeing and/or lightening the hair.

One of the dyeing methods is "permanent" or oxidation dyeing, which uses dye compositions containing oxidation dye precursors, generally known as oxidation bases. These oxidation bases are colourless or weakly coloured compounds, which, when combined with oxidizing products, may give rise to coloured compounds via a process of oxidative condensation.

The processes usually used for dyeing and/or lightening human keratin fibres consist in using (in combination with the dye composition in the case of a dyeing process) an aqueous composition comprising at least one oxidizing agent, under alkaline pH conditions in the vast majority of cases. This oxidizing agent has the role of degrading the melanin of the hair, which, depending on the nature of the oxidizing agent present, leads to more or less pronounced lightening of the fibres. It also has the role of activating the oxidation of the oxidation dye precursors and the formation of coloured species. The oxidizing agent generally used is hydrogen peroxide.

One of the difficulties arises from the fact that the dyeing and/or lightening process is performed under alkaline conditions and that the alkaline agent most commonly used is aqueous ammonia (or ammonium hydroxide). The use of aqueous ammonia is particularly advantageous in processes of this type. Specifically, it enables adjustment of the pH of the composition to an alkaline pH in order to allow activation of the oxidizing agent. This basifying agent also brings about swelling of the keratin fibre, with raising of the scales, which promotes the penetration of the oxidizing agent and of the oxidation dyes into the fibre and thus increases the efficacy of the dyeing and/or lightening reactions.

However, this basifying agent is highly volatile, and this causes unpleasantness to the user on account of the strong and fairly unpleasant characteristic odour of ammonia that is given off during the process.

Furthermore, the amount of ammonia given off requires the use of higher contents than necessary in order to compensate for this loss. This is not without consequences for the user, who not only remains inconvenienced by the odour, but may also be confronted with greater risks of intolerance, for instance irritation of the scalp, which is reflected especially by stinging.

It has been proposed to replace all or some of the aqueous ammonia with one or more other standard basifying agents, but the solutions proposed hitherto do not result in compositions that are as effective as those based on aqueous ammonia, especially since these basifying agents do not provide sufficient lightening or dyeing of the pigmented fibres in the presence of the oxidizing agent.

Now, oxidation dyeing must satisfy a certain number of requirements. Thus, it must be free of toxicological drawbacks, it must enable varied shades to be obtained which have good resistance to external attacking factors such as light, bad weather, washing, permanent waving, perspiration and rubbing.

The colourings must also be powerful and be able to cover grey hair and, finally, they must be as unselective as possible, i.e. they must produce the smallest possible colour differences along the same keratin fibre, which generally comprises areas that are differently sensitized (i.e. damaged) from its end to its root.

The compositions obtained must also have good mixing and application properties, and in particular good rheological properties so as not to run down onto the face, the scalp or beyond the areas that it is proposed to dye, when they are applied.

Finally, the dyeing operations must, as far as is possible, respect the integrity of the keratin fibres and give said fibres the best possible cosmetic properties.

Many attempts have been made in the field of hair dyeing in order to improve the dyeing properties, for example with the aid of adjuvants. For example FR 3 015 240 A1 explores the possibility to provide hair colouring or discolouration compositions with low selectivity and reduced odour and irritation. A mixture of guanidine carbonate with ammonium hydroxide is used as alkaline agent. In addition, cationic polymers are added to the compositions, specifically hexamdimethrin chloride and polyquaternium-6.

However, the choice of these adjuvants is difficult in so far as they must improve the dyeing properties of dye compositions without harming the other properties of these compositions. In particular, these adjuvants must not harm the stability of the compositions, the application properties of the dyeing operation or the cosmetic properties of the dyed fibres.

In recent years, dyeing and/or lightening products comprising a large content of oil (for example greater than 20%) have been developed to improve the colour rendition and the quality of the hair after dyeing. However, the use of such an amount of oil may prove to be unfavourable from the point of view of the cost of producing the formulations.

Moreover, these large amounts of oils penalize the ease of removal of the products on rinsing with water.

Thus, one of the objectives of the present invention is to propose compositions for dyeing and/or lightening human keratin fibres such as the hair, which do not have the drawbacks mentioned above, i.e. which are capable of providing very good dyeing and/or lightening performance qualities while at the same time having working qualities that are superior to those of the existing compositions, especially by having a less disagreeable odour during their application to the fibres or during their preparation, good comfort of the scalp and better ease of removal, and which are more advantageous from an economic viewpoint.

These aims and others are achieved by the present invention, one subject of which is thus a cosmetic composition in particular for dyeing and/or lightening keratin fibres, in particular human keratin fibres such as the hair, comprising:

(a) one or more guanidine salts,
(b) one or more alkanolamine(s),
(c) ammonium hydroxide and
(d) at least two polymers chosen from cationic and amphoteric polymers, which are different from each other, the cationic polymers being chosen from

(1) Homopolymers or copolymers derived from acrylic or methacrylic esters or amides and comprising at least one of the units of formula (I), (II), (III) or (IV) below:

(I) 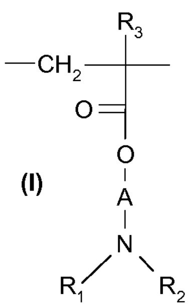 (II) 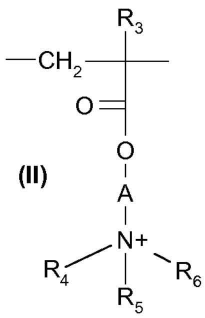 (III) 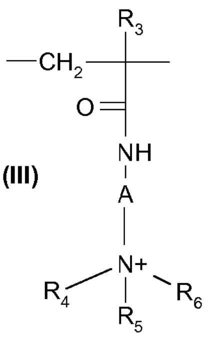 (IV) 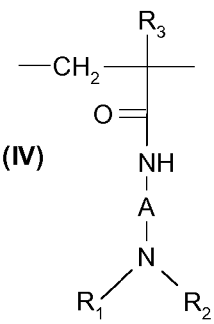

in which:

$R_3$, which may be identical or different, denote a hydrogen atom or a $CH_3$ radical;
A, which may be identical or different, represent a linear or branched $C_1$-$C_6$ and preferably $C_2$-$C_3$ alkyl group or a $C_1$-$C_4$ hydroxyalkyl group;
$R_4$, $R_5$ and $R_6$, which may be identical or different, represent a $C_1$-$C_{18}$ alkyl group or a benzyl radical, and preferably a $C_1$-$C_6$ alkyl group;
$R_1$ and $R_2$, which may be identical or different, represent hydrogen or a $C_1$-$C_6$ alkyl group, preferably methyl or ethyl;
X denotes an anion derived from a mineral or organic acid, such as a methosulfate anion or a halide such as chloride or bromide,

(2) Cationic cellulose derivatives such as cellulose copolymers or cellulose derivatives grafted with a water-soluble quaternary ammonium monomer,
(3) Cationic guar gums ,
(4) Polymers constituted of piperazinyl units and of divalent alkylene or hydroxyalkylene radicals containing straight or branched chains, optionally interrupted with oxygen, sulfur or nitrogen atoms or by aromatic or heterocyclic rings, and also the oxidation and/or quaternization products of these polymers,
(5) Water-soluble polyaminoamides ,
(6) Polymers obtained by reaction of a polyalkylene polyamine containing two primary amine groups and at least one secondary amine group with a dicarboxylic acid chosen from diglycolic acid and saturated $C_3$-$C_8$ aliphatic dicarboxylic acids. ,
(7) Cyclopolymers of alkyldiallylamine or of dialkyldiallylammonium, such as the homopolymers or copolymers containing, as main constituent of the chain, units corresponding to formula (V) or (VI):

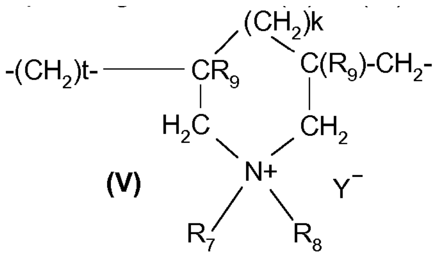

(V)

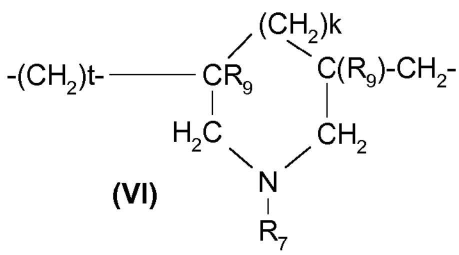

(VI)

in which formulae k and t are equal to 0 or 1, the sum k + t being equal to 1; Rg denotes a hydrogen atom or a methyl radical; $R_7$ and $R_8$, independently of each other, denote a $C_1$-$C_8$ alkyl group, a hydroxyalkyl group in which the alkyl group is $C_1$-$C_5$, an amidoalkyl group in which the alkyl is $C_1$-$C_4$; $R_7$ and $R_8$ can also denote, together with the nitrogen atom to which they are attached, a heterocyclic group such as piperidyl or morpholinyl; $R_7$ and $R_8$, independently of each other, preferably denote a $C_1$-$C_4$ alkyl group; $Y^-$ is an organic or mineral anion such as bromide, chloride, acetate, borate, citrate, tartrate, bisulfate, bisulfite, sulfate or phosphate,

(8) The quaternary diammonium polymer containing repeating units corresponding to the formula:

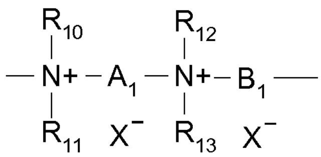

(VII)

in which formula:

$R_{10}$, $R_{11}$, $R_{12}$ and $R_{13}$, which may be identical or different, represent $C_1$-$C_{20}$ aliphatic, alicyclic or arylaliphatic radicals or hydroxyalkylaliphatic radicals in which the alkyl radical is $C_1$-$C_4$, or alternatively $R_{10}$, $R_{11}$, $R_{12}$ and $R_{13}$, together or separately, constitute, with the nitrogen atoms to which they are attached, heterocycles optionally containing a second heteroatom other than nitrogen, or alternatively $R_{10}$, $R_{11}$, $R_{12}$ and $R_{13}$ represent a linear or branched $C_1$-$C_6$ alkyl radical substituted with a nitrile, ester, acyl or amide group or a group -CO-O-$R_{14}$-D or -CO-NH-$R_{14}$-D where $R_{14}$ is an alkylene and D is a quaternary ammonium group;

$A_1$ and $B_1$ represent linear or branched, saturated or unsaturated $C_2$-$C_{20}$ polymethylene groups which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and

$X^-$ denotes an anion derived from a mineral or organic acid;

$A_1$, $R_{10}$ and $R_{12}$ can form, with the two nitrogen atoms to which they are attached, a piperazine ring;

in addition, if $A_1$ denotes a linear or branched, saturated or unsaturated alkylene or hydroxyalkylene radical, $B_1$ may also denote a group $-(CH_2)_n$-CO-D-OC-$(CH_2)_n$- in which n is between 1 and 100 and preferably between 1 and 50, and D denotes:

a) a glycol residue of formula: -O-Z-O-, where Z denotes a linear or branched hydrocarbon-based radical or a group corresponding to one of the following formulae:
$-(CH_2-CH_2-O)_x-CH_2-CH_2-$ and $-[CH_2-CH(CH_3)-O]_y-CH_2-CH(CH_3)-$ where x and y denote an integer from 1 to 4, representing a defined and unique degree of polymerization or any number from 1 to 4 representing an average degree of polymerization;
b) a bis-secondary diamine residue, such as a piperazine derivative;
c) a bis-primary diamine residue of formula: -NH-Y-NH-, where Y denotes a linear or branched hydrocarbon-based radical, or alternatively the radical $-CH_2-CH_2-S-S-CH_2$
$-CH_2-$;
d) a ureylene group of formula: -NH-CO-NH-.
Preferably, $X^-$ is an anion, such as chloride or bromide,

(9) Polyquaternary ammonium polymers constituted of repeating units of formula (IX):

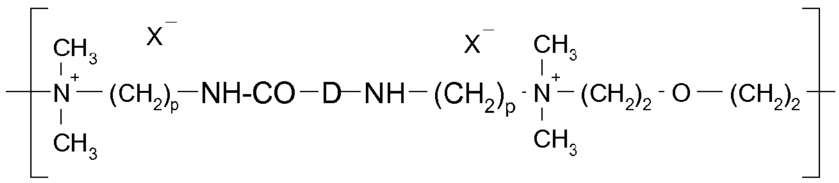

**(IX)**

in which p denotes an integer ranging from 1 to 6 approximately, D may be zero or may represent a group $-(CH_2)_r-CO-$ in which r denotes a number equal to 4 or 7, and $X^-$ is an anion,
(10) Quaternary polymers of vinylpyrrolidone and of vinylimidazole,
(11) Polyamines,
and mixtures thereof,

(e) optionally one or more colouring agents.

A subject of the present invention is also a process for dyeing and/or lightening keratin fibres, in particular human keratin fibres such as the hair, in which the dyeing and/or lightening composition according to the invention is applied to said fibres.

The invention also relates to a multi-compartment device for using the composition according to the invention.

The compositions according to the invention thus provide very good performance qualities in terms of lightening keratin fibres.

Moreover, when they comprise oxidation dyes, the compositions according to the invention thus make it possible to give good performance qualities in terms of dyeing keratin fibres, in particular colourings that are powerful, intense, chromatic and/or sparingly selective, i.e. colourings that are uniform along the fibre.

Moreover, the dyeing and/or lightening process according to the invention also allows the use of compositions that are less malodorous during their application to keratin fibres or during their preparation.

The composition according to the invention is stable over time and has good working qualities on heads, and in particular is easy to use, does not run and allows uniform spreading on the hair. It is easily removed on rinsing.

In addition, the compositions according to the invention are comfortable on the scalp when compared with the existing lightening compositions. Moreover, the compositions according to the invention satisfactorily respect the integrity of the keratin fibres on conclusion of the dyeing and/or lightening process.

Other characteristics and advantages of the invention will emerge more clearly on reading the description and the examples that follow.

In the text hereinbelow, and unless otherwise indicated, the limits of a range of values are included within that range.

The human keratin fibres treated via the process according to the invention are preferably the hair.

The expression *"at least one"* is equivalent to the expression *"one or more".*

### *(a) Guanidine salt*

The composition according to the invention comprises one or more guanidine salts. The total content of guanidine salt(s) may range, for example, from 0.1 % to 15% by weight, preferably from 0.5% to 10% by weight and better still from 1% to 8% by weight relative to the total weight of the composition.

The guanidine salt may be chosen from organic or inorganic guanidine salts. The organic salts are chosen from the salts of organic acids, such as citrates, lactates, glycolates, gluconates, acetates, propionates, fumarates, oxalates and tartrates.

Preferably, the inorganic salts are chosen from halides, hydrohalides (for example hydrochlorides), carbonate, hydrogen carbonate, sulfate, nitrate, sulfamate, and guanidine phosphates such as monoguanidine phosphate and diguanidine phosphate.

Preferably, the guanidine salts are chosen from inorganic guanidine salts, in particular guanidine chloride or hydrochloride, guanidine carbonate or hydrogen carbonate, guanidine phosphates such as monoguanidine phosphate and diguanidine phosphate, or guanidine sulfamate. Even more preferentially, the guanidine salt is guanidine carbonate or guanidine hydrogen carbonate. Better still, the guanidine salt is guanidine carbonate.

### *(b) Alkanolamine*

The term "alkanolamine" means an organic amine comprising a primary, secondary or tertiary amine function, and one or more linear or branched $C_1$-$C_8$ alkyl groups bearing one or more hydroxyl radicals.

Alkanolamines such as monoalkanolamines, dialkanolamines or trialkanolamines comprising from one to three identical or different $C_1$-$C_4$ hydroxyalkyl radicals are in particular suitable for performing the invention.

Among compounds of this type, mention may be made of monoethanolamine, diethanolamine, triethanolamine, monoisopropanolamine, diisopropanolamine, N-dimethylaminoethanolamine, 2-amino-2-methyl-1-propanol, triisopropanolamine, 2-amino-2-methyl-1,3-propanediol, 3-amino-1,2-propanediol, 3-dimethylamino-1,2-propanediol and tris(hydroxymethylamino)methane. Preferentially, the alkanolamine is monoethanolamine.

The composition according to the invention generally comprises a total content of alkanolamine(s) ranging from 0.01% to 10% by weight, preferably from 0.1% to 7% by weight and better still from 0.5% to 5% by weight relative to the weight of said composition.

***(c) Ammonium hydroxide***

The content of ammonium hydroxide in the composition according to the invention more particularly represents from 0.01% to 10% by weight, preferably from 0.1% to 10% by weight and more preferentially from 0.5% to 8% by weight relative to the total weight of the composition.

Preferably, the guanidine carbonate/(alkanolamines + ammonium hydroxide) weight ratio in the composition according to the invention is less than or equal to 1, preferably less than or equal to 0.9. It may especially range from 0.1 to 1 and better still from 0.2 to 0.9. Preferably, the guanidine carbonate/(monoethanolamine + ammonium hydroxide) weight ratio is less than or equal to 1, preferably less than or equal to 0.9. It may especially range from 0.1 to 1 and better still from 0.2 to 0.9.

*Additional alkaline agent*

According to one embodiment, the composition according to the invention may also comprise at least one additional alkaline agent other than the guanidine salts, the alkanolamines and ammonium hydroxide, which may be a Brønsted-Lowry or Lewis base. It may be mineral or organic.

In particular, the additional alkaline agent(s) may be chosen from:

oxyethylenated and/or oxypropylenated ethylenediamines,
inorganic or organic hydroxides,
alkali metal silicates, such as sodium metasilicates,
amino acids, preferably basic amino acids, such as arginine, lysine, ornithine, citrulline and histidine,
carbonates and bicarbonates, particularly of a primary amine, secondary amine or tertiary amine, of an alkali metal or alkaline-earth metal, or of ammonium, and
the compounds of formula (Q) below:

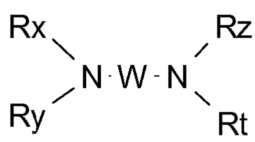
(Q)

in which W is a $C_1$-$C_6$ alkylene residue optionally substituted with a hydroxyl group or a $C_1$-$C_6$ alkyl group; Rx, Ry, Rz and Rt, which may be identical or different, represent a hydrogen atom or a $C_1$-$C_6$ alkyl, $C_1$-$C_6$ hydroxyalkyl or $C_1$-$C_6$ aminoalkyl group, and mixtures thereof.

Examples of such compounds of formula (Q) that may be mentioned include 1,3-diaminopropane, 1,3-diamino-2-propanol, spermine and spermidine.

The mineral or organic hydroxides are preferably chosen from hydroxides of an alkali metal, hydroxides of an alkaline-earth metal, for instance sodium hydroxide or potassium hydroxide, hydroxides of a transition metal, such as hydroxides of metals from Groups III, IV, V and VI of the Periodic Table of the Elements, and hydroxides of lanthanides or actinides.

The additional alkaline agent(s) may be present in the lightening composition according to the invention in a content ranging from 0.05% to 5% by weight, preferably from 0.1% to 3% by weight and better still from 0.2% to 2% by weight relative to the total weight of the composition.

According to one embodiment, the composition according to the invention is free of additional alkaline agents other than the guanidine salt(s), the alkanolamine(s) and ammonium hydroxide.

Preferably, the composition according to the invention is free of additional alkaline agents other than the guanidine salt(s), the alkanolamine(s) and ammonium hydroxide.

***(d) Cationic and amphoteric polymers***

The composition according to the invention comprises at least two polymers chosen from cationic and amphoteric polymers, which are different from each other.

*Cationic polymer*

It is recalled that, for the purposes of the present invention, the term "cationic polymer" denotes any polymer containing cationic groups and/or groups that can be ionized into cationic groups and not containing any anionic groups and/or groups that can be ionized into anionic groups.

Preferably, the cationic polymers present in the composition are linear, random, grafted or block homopolymers or copolymers and comprise at least one cationic group and/or group that can be ionized into a cationic group chosen from primary, secondary, tertiary and/or quaternary amine groups that form part of the main polymer chain or that are borne by a side substituent directly connected thereto.

Preferably, the cationic charge density of the cationic polymers according to the invention is greater than 1 meq/g and even more advantageously greater than or equal to 4 meq/g.

This charge density is determined by the Kjeldahl method. It may also be calculated from the chemical nature of the polymer.

The cationic polymers used generally have a number-average molecular weight of between 500 and $5 \times 10^6$ approximately, and preferably between $10^3$ and $3 \times 10^6$.

The cationic polymer(s) is (are) chosen from the following polymers, alone or as a mixture:

(1) Homopolymers or copolymers derived from acrylic or methacrylic esters or amides and comprising at least one of the units of formula (I), (II), (III) or (IV) below:

(I) 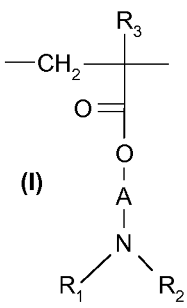 (II) 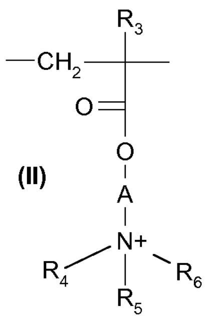 (III) 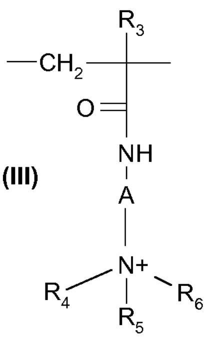 (IV) 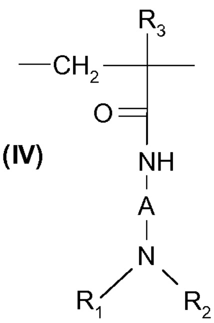

in which:

$R_3$, which may be identical or different, denote a hydrogen atom or a $CH_3$ radical;
A, which may be identical or different, represent a linear or branched $C_1$-$C_6$ and preferably $C_2$-$C_3$ alkyl group or a $C_1$-$C_4$ hydroxyalkyl group;
$R_4$, $R_5$ and $R_6$, which may be identical or different, represent a $C_1$-$C_{18}$ alkyl group or a benzyl radical, and preferably a $C_1$-$C_6$ alkyl group;
$R_1$ and $R_2$, which may be identical or different, represent hydrogen or a $C_1$-$C_6$ alkyl group, preferably methyl or ethyl;
X denotes an anion derived from a mineral or organic acid, such as a methosulfate anion or a halide such as chloride or bromide.

The polymers of family

(1) may also contain one or more units derived from comonomers which may be chosen from the family of acrylamides, methacrylamides, diacetone acrylamides, acrylamides and methacrylamides substituted on the nitrogen with lower ($C_1$-$C_4$) alkyls, acrylic or methacrylic acids or esters thereof, vinyllactams such as vinylpyrrolidone or vinylcaprolactam, and vinyl esters.
Thus, among these polymers of family (1), mention may be made of:

- copolymers of acrylamide and of dimethylaminoethyl methacrylate quaternized with dimethyl sulfate or with a dimethyl halide, such as the product sold under the name Hercofloc by the company Hercules,
- the copolymers of acrylamide and of methacryloyloxyethyl-trimethylammonium chloride described, for example, in EP 80 976 and sold under the name Bina Quat P 100 by the company Ciba Geigy,
- the copolymer of acrylamide and of methacryloyloxyethyltrimethylammonium methosulfate sold under the name Reten by the company Hercules,
- quaternized or non-quaternized vinylpyrrolidone/dialkylaminoalkyl acrylate or methacrylate copolymers, such as the products sold under the name Gafquat by the company ISP, for instance Gafquat 734 or Gafquat 755, or alternatively the products known as Copolymer 845, 958 and 937. These polymers are described in FR 2 077 143 and FR 2 393 573,
- dimethylaminoethyl methacrylate/vinylcaprolactam/vinylpyrrolidone terpolymers, such as the product sold under the name Gaffix VC 713 by the company ISP,
- vinylpyrrolidone/methacrylamidopropyldimethylamine copolymers sold in particular under the name Styleze CC 10 by ISP,
- quaternized vinylpyrrolidone/dimethylaminopropylmethacrylamide copolymers such as the product sold under the name Gafquat HS 100 by the company ISP, and
- the crosslinked polymers of methacryloyloxy($C_1$-$C_4$)alkyl tri($C_1$-$C_4$)alkylammonium salts, such as the polymers obtained by homopolymerization of dimethylaminoethyl methacrylate quaternized with methyl chloride, or by copolymerization of acrylamide with dimethylaminoethyl methacrylate quaternized with methyl chloride, the homo- or copolymerization being followed by crosslinking with an olefinically unsaturated compound, more particularly methylenebisacrylamide. Use may more particularly be made of a crosslinked acrylamide/methacryloyloxyethyltrimethylammonium chloride copolymer (20/80 by weight) in the form of a dispersion containing 50% by weight of said copolymer in mineral oil. This dispersion is sold under the name Salcare® SC 92 by the company Ciba. Use may also be made of a crosslinked homopolymer of methacryloyloxyethyltrimethylammonium chloride comprising approximately 50% by weight of the homopolymer in mineral oil or in a liquid ester. These dispersions are sold under the names Salcare® SC 95 and Salcare® SC 96 by the company Ciba.

(2) Cationic cellulose derivatives such as cellulose copolymers or cellulose derivatives grafted with a water-soluble quaternary ammonium monomer, and disclosed in particular in US 4 131 576, such as hydroxyalkyl celluloses, for instance hydroxymethyl, hydroxyethyl or hydroxypropyl celluloses grafted in particular with a methacryloylethyltrimethylammonium, methacrylamidopropyltrimethylammonium or dimethyldiallylammonium salt.
The commercial products corresponding to this definition are more particularly the products sold under the names Celquat L 200 and Celquat H 100 by the company National Starch.
(3) Cationic guar gums described more particularly in US 3 589 578 and US 4 031 307, such as guar gums containing trialkylammonium cationic groups. Use is made, for example, of guar gums modified with a 2,3-epoxypropyltrimethylammonium salt (for example, chloride).
Such products are sold in particular under the trade names Jaguar C13S, Jaguar C15, Jaguar C17 and Jaguar C162 by the company Meyhall.
(4) Polymers constituted of piperazinyl units and of divalent alkylene or hydroxyalkylene radicals containing straight or branched chains, optionally interrupted with oxygen, sulfur or nitrogen atoms or by aromatic or heterocyclic rings, and also the oxidation and/or quaternization products of these polymers. Such polymers are described, in particular, in FR 2 162 025 and FR 2 280 361.
(5) Water-soluble polyaminoamides prepared in particular by polycondensation of an acidic compound with a polyamine; these polyaminoamides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyaminoamide; these polyaminoamides may be alkylated or, if they contain one or more tertiary amine functions, they may be quaternized. Such polymers are described, in particular, in FR 2 252 840 and FR 2 368 508.
Polyaminoamide derivatives resulting from the condensation of polyalkylene polyamines with polycarboxylic acids, followed by an alkylation with difunctional agents. Mention may be made, for example, of adipic acid/dialkylaminohydroxyalkyldialkylene-triamine polymers in which the alkyl radical is $C_1$-$C_4$ and preferably denotes methyl, ethyl or propyl. Such polymers are described in particular in FR 1 583 363.
Among these derivatives, mention may be made more particularly of the adipic acid/dimethylaminohydroxypropyl/diethylenetriamine polymers sold under the name Cartaretine F, F4 or F8 by the company Sandoz.
(6) Polymers obtained by reaction of a polyalkylene polyamine containing two primary amine groups and at least

one secondary amine group with a dicarboxylic acid chosen from diglycolic acid and saturated $C_3$-$C_8$ aliphatic dicarboxylic acids. The mole ratio between the polyalkylene polyamine and the dicarboxylic acid is between 0.8:1 and 1.4:1; the resulting polyaminoamide is reacted with epichlorohydrin in a mole ratio of epichlorohydrin relative to the secondary amine group of the polyaminoamide of between 0.5:1 and 1.8:1. Such polymers are described in particular in US 3 227 615 and US 2 961 347.

Polymers of this type are sold in particular under the name Hercosett 57, PD 170 or Delsette 101 by the company Hercules.

(7) Cyclopolymers of alkyldiallylamine or of dialkyldiallylammonium, such as the homopolymers or copolymers containing, as main constituent of the chain, units corresponding to formula (V) or (VI):

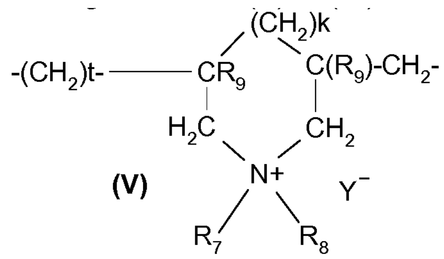
(V)

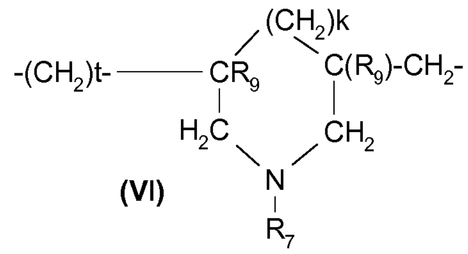
(VI)

in which formulae k and t are equal to 0 or 1, the sum k + t being equal to 1; Rg denotes a hydrogen atom or a methyl radical; $R_7$ and $R_8$, independently of each other, denote a $C_1$-$C_8$ alkyl group, a hydroxyalkyl group in which the alkyl group is $C_1$-$C_5$, an amidoalkyl group in which the alkyl is $C_1$-$C_4$; $R_7$ and $R_8$ can also denote, together with the nitrogen atom to which they are attached, a heterocyclic group such as piperidyl or morpholinyl; $R_7$ and $R_8$, independently of each other, preferably denote a $C_1$-$C_4$ alkyl group; $Y^-$ is an organic or mineral anion such as bromide, chloride, acetate, borate, citrate, tartrate, bisulfate, bisulfite, sulfate or phosphate. These polymers are described in particular in FR 2 080 759 and FR 2 190 406.

The cyclopolymers preferably comprise at least one unit of formula (V).

As regards the copolymers, they also comprise an acrylamide monomer.

Among the polymers defined above, mention may be made more particularly of the dimethyldiallylammonium chloride homopolymer sold under the name Merquat 100 by the company Nalco (and its homologues of low weight-average molecular mass) and the copolymers of diallyldimethylammonium chloride and of acrylamide, sold under the name Merquat 550.

(8) The quaternary diammonium polymer containing repeating units corresponding to the formula:

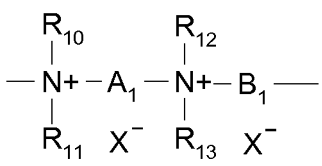
(VII)

in which formula:

$R_{10}$, $R_{11}$, $R_{12}$ and $R_{13}$, which may be identical or different, represent $C_1$-$C_{20}$ aliphatic, alicyclic or arylaliphatic radicals or hydroxyalkylaliphatic radicals in which the alkyl radical is $C_1$-$C_4$, or alternatively $R_{10}$, $R_{11}$, $R_{12}$ and $R_{13}$, together or separately, constitute, with the nitrogen atoms to which they are attached, heterocycles optionally containing a second heteroatom other than nitrogen, or alternatively $R_{10}$, $R_{11}$, $R_{12}$ and $R_{13}$ represent a linear or branched $C_1$-$C_6$ alkyl radical substituted with a nitrile, ester, acyl or amide group or a group -CO-O-$R_{14}$-D or -CO-NH-$R_{14}$-D where $R_{14}$ is an alkylene and D is a quaternary ammonium group;

$A_1$ and $B_1$ represent linear or branched, saturated or unsaturated $C_2$-$C_{20}$ polymethylene groups which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and

$X^-$ denotes an anion derived from a mineral or organic acid;

$A_1$, $R_{10}$ and $R_{12}$ can form, with the two nitrogen atoms to which they are attached, a piperazine ring;

in addition, if $A_1$ denotes a linear or branched, saturated or unsaturated alkylene or hydroxyalkylene radical, $B_1$ may also denote a group $-(CH_2)_n$-CO-D-OC-$(CH_2)_n$- in which n is between 1 and 100 and preferably between 1 and 50, and D denotes:

a) a glycol residue of formula: -O-Z-O-, where Z denotes a linear or branched hydrocarbon-based radical or a group corresponding to one of the following formulae: $-(CH_2-CH_2-O)_x-CH_2-CH_2-$ and $-[CH_2-CH(CH_3)-O]_y-CH_2-CH(CH_3)-$ where x and y denote an integer from 1 to 4, representing a defined and unique degree of polymerization or any number from 1 to 4 representing an average degree of polymerization;
b) a bis-secondary diamine residue, such as a piperazine derivative;
c) a bis-primary diamine residue of formula: -NH-Y-NH-, where Y denotes a linear or branched hydrocarbon-based radical, or alternatively the radical $-CH_2-CH_2-S-S-CH_2-CH_2-$;
d) a ureylene group of formula: -NH-CO-NH-.

Preferably, $X^-$ is an anion, such as chloride or bromide.
These polymers have a number-average molecular weight generally between 1000 and 100 000.
Polymers of this type are described in particular in FR 2 320 330, FR 2 270 846, FR 2 316 271, FR 2 336 434, FR 2 413 907, US 2 273 780, US 2 375 853, US 2 388 614, US 2 454 547, US 3 206 462, US 2 261 002, US 2 271 378, US 3 874 870, US 4 001 432, US 3 929 990, US 3 966 904, US 4 005 193, US 4 025 617, US 4 025 627, US 4 025 653, US 4 026 945 and US 4 027 020. It is more particularly possible to use polymers that are constituted of repeating units corresponding to the following formula (VIII):

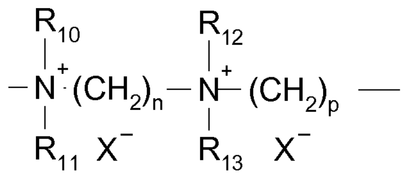

**(VIII)**

in which $R_{10}$, $R_{11}$, $R_{12}$ and $R_{13}$, which may be identical or different, denote a $C_1$-$C_4$ alkyl or hydroxyalkyl radical, n and p are integers ranging from 2 to 20 approximately, and $X^-$ is an anion derived from a mineral or organic acid.

(9) Polyquaternary ammonium polymers constituted of repeating units of formula (IX):

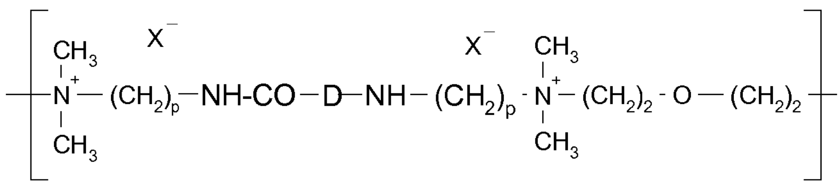

**(IX)**

in which p denotes an integer ranging from 1 to 6 approximately, D may be zero or may represent a group $-(CH_2)_r-CO-$ in which r denotes a number equal to 4 or 7, and $X^-$ is an anion.
Such polymers may be prepared according to the processes described in US 4 157 388, US 4 702 906 and US 4 719 282. They are in particular described in patent application EP 122 324.
Among these polymers, examples that may be mentioned include the products Mirapol A 15, Mirapol AD1, Mirapol AZ1 and Mirapol 175 sold by the company Miranol.
(10) Quaternary polymers of vinylpyrrolidone and of vinylimidazole, for instance the products sold under the names Luviquat FC 905, FC 550 and FC 370 by the company BASF.
(11) Polyamines such as Polyquart H sold by Cognis, referred to under the name polyethylene glycol (15) tallow polyamine in the CTFA dictionary.

Other cationic polymers that may be used in the context of the invention are polyalkyleneimines, in particular polyethyleneimines, polymers containing vinylpyridine or vinylpyridinium units, condensates of polyamines and of epichlorohydrin, polyquaternary ureylenes and chitin derivatives.

Among all the cationic polymers that may be used in the context of the present invention, it is preferred to use, alone or as mixtures, polymers of families (1), (7), (8) and (9). In accordance with a more particular embodiment of the invention, it is preferred to use polymers of families (7), (8) and (9).

According to an even more advantageous embodiment of the invention, use is made of polymers of families (7) and (8) alone or as mixtures, and even more preferentially of polymers bearing repeating units of formulae (W) and (U) below:

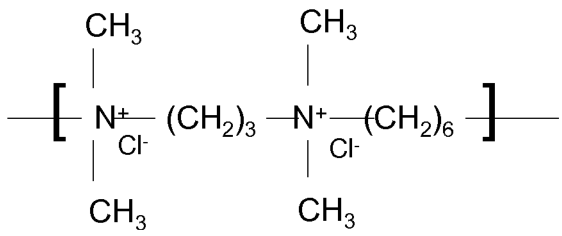

**(W)**

and in particular those of which the molecular weight, determined by gel permeation chromatography, is between 9500 and 9900;

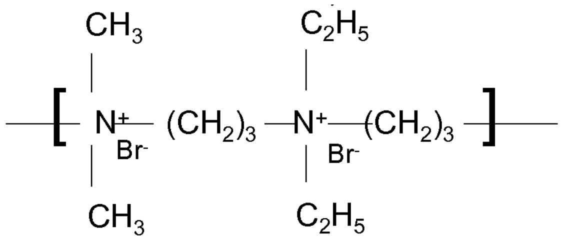

**(U)**

and especially those whose molecular weight, determined by gel permeation chromatography, is about 1200.

Among the polymers bearing repeating units of formula (W), mention may be made of the polymer whose INCI name is hexadimethrine chloride.

*Amphoteric polymer*

It is recalled that, for the purposes of the present invention, the term "amphoteric polymer" denotes any polymer containing cationic groups and/or groups that can be ionized into cationic groups and anionic groups and/or groups that can be ionized into anionic groups. The amphoteric (or zwitterionic) polymers that may be used in accordance with the invention may be selected from polymers comprising units B and C distributed statistically in the polymer chain, where B denotes a unit derived from a monomer comprising at least one basic nitrogen atom and C denotes a unit derived from an acid monomer comprising one or more carboxylic or sulfonic groups, or alternatively B and C may denote groups derived from carboxybetaine or sulfobetaine zwitterionic monomers;
B and C may also denote a cationic polymer chain comprising primary, secondary, tertiary or quaternary amine groups, in which at least one of the amine groups bears a carboxylic or sulfonic group connected via a hydrocarbon-based radical or alternatively B and C form part of a chain of a polymer comprising an $\alpha,\beta$-dicarboxylic ethylene unit in which one of the carboxylic groups has been made to react with a polyamine comprising one or more primary or secondary amine groups.

The amphoteric polymers corresponding to the definition given above that are more particularly preferred are chosen from the following polymers:

(1') polymers comprising as monomers at least one monomer derived from a vinyl compound carrying a carboxyl group, such as, more particularly, acrylic acid, methacrylic acid, maleic acid, alpha-chloroacrylic acid, and at least one basic monomer derived from a substituted vinyl compound containing at least one basic atom, chosen especially from the following:

a) dialkylaminoalkyl methacrylates, dialkylaminoalkyl acrylates, dialkylaminoalkylmethacrylamides and dialkylaminoalkylacrylamides, Such compounds are described in United States patent No 3 836 537,

b) trialkylaminoalkyl methacrylate salts and trialkylaminoalkyl acrylate salts, and salts of trialkylaminoalkylmethacrylamide and of trialkylaminoalkylacrylamide,
Mention may be made especially of the acrylic acid/acrylamidopropyltrimethylammonium chloride copolymer available from the company Stockhausen under the name Polymer W3794. Mention may also be made of the acrylic acid/acrylamidopropyltrimethylammonium chloride/acrylamide copolymers available from the company Nalco under the names Merquat 2001 and Merquat 2003.

(2') polymers comprising units derived from:

a) at least one monomer chosen from acrylamides and methacrylamides substituted on the nitrogen with an alkyl radical,
b) at least one acidic comonomer containing one or more reactive carboxylic groups, and
c) at least one basic comonomer such as esters bearing primary, secondary, tertiary and quaternary amine substituents of acrylic and methacrylic acids and the product of quaternization of dimethylaminoethyl methacrylate with dimethyl or diethyl sulfate.

The N-substituted acrylamides or methacrylamides that are more particularly preferred according to the invention are groups in which the alkyl radicals contain from 2 to 12 carbon atoms and more particularly N-ethylacrylamide, N-tert-butylacrylamide, N-tert-octylacrylamide, N-octylacrylamide, N-decylacrylamide, N-dodecylacrylamide and the corresponding methacrylamides.
The acidic comonomers are more particularly chosen from acrylic, methacrylic, crotonic, itaconic, maleic and fumaric acid and alkyl monoesters, containing 1 to 4 carbon atoms, of maleic or fumaric acid or anhydride.
The preferred basic comonomers are aminoethyl, butylaminoethyl, N,N'-dimethylaminoethyl and N-tert-butylaminoethyl methacrylates.
The copolymers whose CTFA (4th edition, 1991) name is octylacrylamide/acrylates/butylaminoethyl methacrylate copolymer, such as the products sold under the name Amphomer LV by the company National Starch, are particularly used.

(3') copolymers comprising as monomers at least one monomer derived from a vinyl compound bearing a carboxylic group, such as, more particularly, acrylic acid, methacrylic acid, maleic acid, alpha-chloroacrylic acid, and at least one monomer of diallyldialkylammonium salt type, the alkyl groups containing from 1 to 6 carbon atoms. Preferably, the alkyl group is a methyl group.
Among these polymers, copolymers comprising as monomers dimethyldiallylammonium chloride and acrylic acid optionally combined with acrylamide are particularly preferred. Mention may be made in particular of the compounds available from the company Nalco under the names Merquat 280, Merquat 295, Merquat 3330, Merquat 3331 and Merquat 3333.

(4') crosslinked and alkylated polyamino amides partially or totally derived from polyamino amides of general formula:

$$\{CO-R_{10}-CO-Z\} \quad (I)$$

in which $R_{10}$ represents a divalent radical derived from a saturated dicarboxylic acid, a mono- or dicarboxylic aliphatic acid containing an ethylenic double bond, an ester of a lower alkanol containing 1 to 6 carbon atoms of these acids, or a radical derived from the addition of any one of said acids to a bis(primary) or bis(secondary) amine, and Z denotes a radical derived from a bis(primary), mono- or bis(secondary) polyalkylene-polyamine and preferably represents:

a) in proportions of from 60 to 100 mol%, the radical:

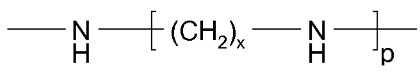 (II)

where x = 2 and p = 2 or 3, or else x = 3 and p = 2,
this radical being derived from diethylenetriamine, triethylenetetramine or dipropylenetriamine;
b) in proportions of from 0 to 40 mol%, the radical (II) above in which x = 2 and p = 1 and which is derived from ethylenediamine, or the radical derived from piperazine:

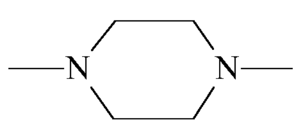

c) in proportions of 0 to 20 mol%, the radical $-NH-(CH_2)_6-NH-$ derived from hexamethylenediamine, these polyaminoamines being crosslinked by addition of a difunctional crosslinking agent chosen from epihalohydrins, diepoxides, dianhydrides, bis-unsaturated derivatives, by means of 0.025 to 0.35 mol of crosslinking agent per

amine group of the polyaminoamide, and being alkylated by the action of acrylic acid, chloroacetic acid or an alkane sultone or salts thereof.

The saturated carboxylic acids are preferably chosen from acids containing from 6 to 10 carbon atoms, such as adipic acid, 2,2,4-trimethyladipic acid and 2,4,4-trimethyladipic acid, terephthalic acid, acids containing an ethylenic double bond, for instance acrylic acid, methacrylic acid and itaconic acid.
The alkane sultones used in the alkylation are preferably propane sultone or butane sultone; the salts of the alkylating agents are preferably the sodium or potassium salts.

(5') polymers comprising zwitterionic units of formula:

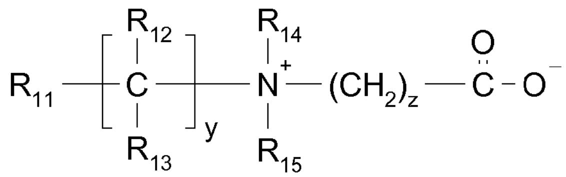
(III)

in which R11 denotes a polymerizable unsaturated group, such as an acrylate, methacrylate, acrylamide or methacrylamide group, y and z represent an integer from 1 to 3, $R_{12}$ and $R_{13}$ represent a hydrogen atom, methyl, ethyl or propyl, and $R_{14}$ and $R_{15}$ represent a hydrogen atom or an alkyl radical such that the sum of the carbon atoms in $R_{14}$ and $R_{15}$ does not exceed 10.
The polymers comprising such units may also comprise units derived from non-zwitterionic monomers such as dimethyl- or diethylaminoethyl acrylate or methacrylate or alkyl acrylates or methacrylates, acrylamides or methacrylamides or vinyl acetate.
Mention may be made, by way of example, of methyl methacrylate/methyl dimethylcarboxymethylammonioethyl methacrylate copolymer, such as the product sold under the name Diaformer Z301 by the company Sandoz.

(6') polymers derived from chitosan comprising monomer units corresponding to the following formulae:

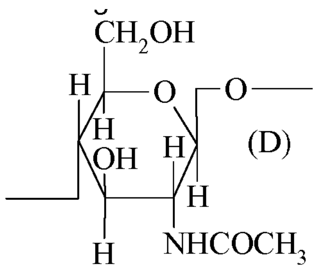
(D)
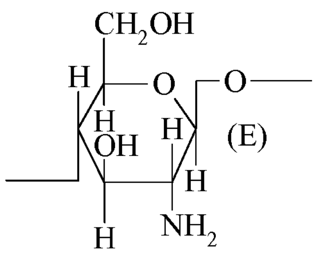
(E)
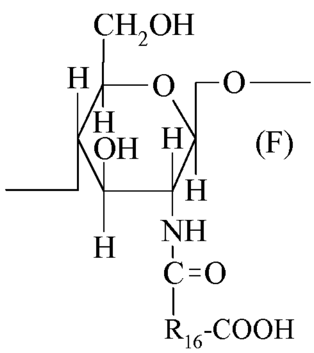
(F)

unit D being present in proportions of between 0 and 30%, unit E in proportions of between 5% and 50% and unit F in proportions of between 30% and 90%, it being understood that, in this unit F, $R_{16}$ represents a radical of formula:

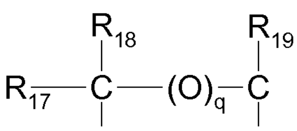

in which, if q = 0, $R_{17}$, $R_{18}$ and $R_{19}$, which may be identical or different, each represent a hydrogen atom, a methyl, hydroxyl, acetoxy or amino residue, a monoalkylamine residue or a dialkylamine residue that are optionally interrupted with one or more nitrogen atoms and/or optionally substituted with one or more amine, hydroxyl, carboxyl, alkylthio or sulfonic groups, an alkylthio residue in which the alkyl group bears an amino residue, at least one of the radicals $R_{17}$, $R_{18}$ and $R_{19}$ being, in this case, a hydrogen atom; or, if q = 1, $R_{17}$, $R_{18}$ and $R_{19}$ each represent a hydrogen atom, and also the salts formed by these compounds with bases or acids.

(7') polymers derived from the N-carboxyalkylation of chitosan, such as N-carboxymethyl chitosan or N-carboxybutyl chitosan, available under the name Evalsan powder from the company Jan Dekker.

(8') polymers containing units corresponding to the general formula (IV) are described in French patent 1 400 366:

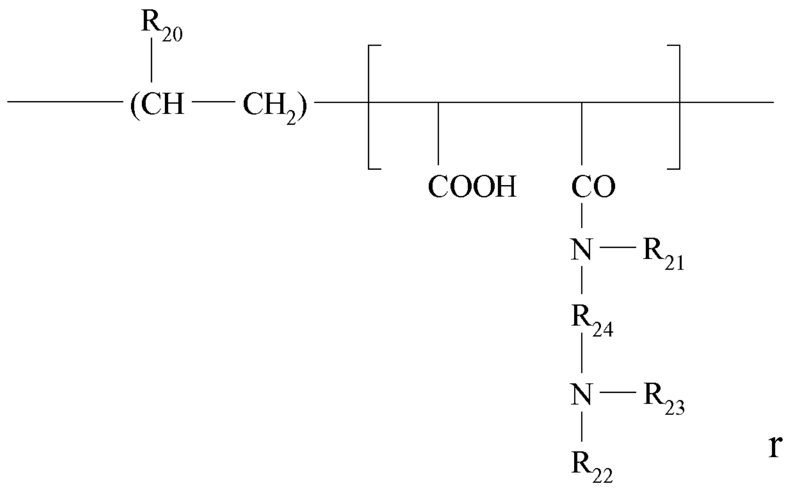

(IV)

in which $R_{20}$ represents a hydrogen atom, a $CH_3O$, $CH_3CH_2O$ or phenyl radical, $R_{21}$ denotes hydrogen or a lower alkyl radical such as methyl or ethyl, $R_{22}$ denotes hydrogen or a lower alkyl radical such as methyl or ethyl, $R_{23}$ denotes a lower alkyl radical such as methyl or ethyl or a radical corresponding to the formula: $-R_{24}-N(R_{22})_2$, $R_{24}$ representing a group $-CH_2-CH_2-$, $-CH_2-CH_2-CH_2-$ or $-CH_2-CH(CH_3)-$, $R_{22}$ having the meanings mentioned above, and also the higher homologues of these radicals, and containing up to 6 carbon atoms.

(9') amphoteric polymers of the -D-X-D-X type chosen from:

a) polymers obtained by the action of chloroacetic acid or sodium chloroacetate on compounds comprising at least one unit of formula:

-D-X-D-X-D- (V)

where D denotes a radical

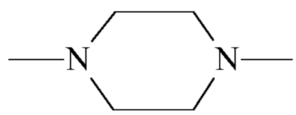

and X denotes the symbol E or E', E or E', which may be identical or different, denote a divalent radical that is an alkylene radical with a straight or branched chain containing up to 7 carbon atoms in the main chain, which is unsubstituted or substituted with hydroxyl groups and which can comprise, in addition to the oxygen, nitrogen and sulfur atoms, 1 to 3 aromatic and/or heterocyclic rings; the oxygen, nitrogen and sulfur atoms being present in the form of ether, thioether, sulfoxide, sulfone, sulfonium, alkylamine or alkenylamine groups, hydroxyl, benzylamine, amine oxide, quaternary ammonium, amide, imide, alcohol, ester and/or urethane groups.

b) polymers of formula:

-D-X-D-X- (VI)

where D denotes a radical

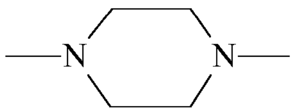

and X denotes the symbol E or E' and at least once E'; E having the meaning given above and E' being a divalent radical that is an alkylene radical bearing a straight or branched chain containing up to 7 carbon atoms in the main chain, which is unsubstituted or substituted with one or more hydroxyl radicals and comprising one or more nitrogen atoms, the nitrogen atom being substituted with an alkyl chain that is optionally interrupted with an oxygen atom and necessarily comprising one or more carboxyl functions or one or more hydroxyl functions and betainized by reaction with chloroacetic acid or sodium chloroacetate.

(10') ($C_1$-$C_5$)alkyl vinyl ether/maleic anhydride copolymers partially modified by semiamidation with an N,N-dialkylaminoalkylamine, such as N,N-dimethylaminopropylamine, or by semiesterification with an N,N-dialkanolamine. These copolymers may also comprise other vinyl comonomers, such as vinylcaprolactam, and mixtures thereof.

The amphoteric polymers that are particularly preferred according to the invention are those of families (1') and (3').
Mention may be made in particular of amphoteric polymers chosen from acrylic acid/acrylamidopropyltrimethylammonium chloride copolymers, acrylic acid/acrylamidopropyltrimethylammonium chloride/acrylamide copolymers, copolymers comprising as monomers dimethyldiallylammonium chloride and acrylic acid optionally combined with acrylamide, and mixtures thereof.

The amphoteric polymers of family (1') will be most particularly preferred, and among these the acrylic acid/acrylamidopropyltrimethylammonium chloride copolymer.

According to one embodiment, the composition comprises at least one cationic polymer and at least one amphoteric polymer, said polymers being chosen from those mentioned above.

According to another embodiment, the composition comprises at least two cationic polymers that are different from each other, chosen from those mentioned above.

In a preferred variant of the invention, the composition comprises at least one cationic polymer chosen from the polymers of family (7) and at least one cationic polymer chosen from the polymers of family (8), in particular polymers bearing repeating units (W) or (U).

In an even more preferential variant, the composition comprises at least one cationic polymer chosen from dimethyldiallylammonium chloride homopolymers and diallyldimethylammonium chloride copolymers, preferably from dimethyldiallylammonium chloride homopolymers, and at least one polymer chosen from polymers bearing repeating units of formula (W), in particular the polymer whose INCI name is hexadimethrine chloride.

In the composition of the invention, the total content of amphoteric and/or cationic polymers preferably represents from 0.01% to 15%, better still from 0.05% to 10% and even more preferentially from 0.1% to 5% by weight relative to the total weight of the composition.

## ***Oxidizing agent***

According to a particular embodiment of the invention, the composition according to the invention also comprises at least one chemical oxidizing agent.

The term "chemical oxidizing agent" means an oxidizing agent other than atmospheric oxygen.

In particular, the chemical oxidizing agent(s) are chosen, for example, from hydrogen peroxide, urea peroxide, alkali metal bromates, peroxygenated salts, for instance persulfates or perborates, peracids and precursors thereof and alkali metal or alkaline-earth metal percarbonates. Advantageously, the oxidizing agent is hydrogen peroxide.

When the oxidizing agent(s) are present in the composition according to the invention, they generally represent a total content ranging from 0.1% to 50% by weight, preferably from 0.5% to 20% by weight and better still from 1% to 15% by weight relative to the total weight of the dye composition.

According to a preferred embodiment, the dye composition according to the invention does not comprise any oxidizing agent. The oxidizing agent is then provided by an oxidizing composition used with the composition according to the invention.

## ***(e) Colouring agent***

The composition may comprise at least one colouring agent, which may be chosen from oxidation dye precursors and direct dyes, and mixtures thereof.

Oxidation dye precursors that may be mentioned include oxidation bases and/or couplers. Preferably, the composition comprises at least one oxidation dye chosen from oxidation bases and couplers, and mixtures thereof.

By way of example, the oxidation bases are chosen from para-phenylenediamines, bis(phenyl)alkylenediamines, para-aminophenols, ortho-aminophenols and heterocyclic bases, and addition salts thereof.

Among the para-phenylenediamines, examples that may be mentioned include para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-N,N-bis(β-hydroxyethyl)amino-2-methylaniline, 4-N,N-bis(β-hydroxyethyl)amino-2-chloroaniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine, 2-β-acetylaminoethyloxy-para-phenylenediamine, N-(β-methoxyethyl)-para-phenylenediamine, 4-aminophenylpyrrolidine, 2-thienyl-para-phenylenediamine, 2-β-hydroxyethylamino-5-aminotoluene, 3-hydroxy-1-(4'-aminophenyl)pyrrolidine, and the addition salts thereof with an acid.

Among the para-phenylenediamines mentioned above, para-phenylenediamine, para-tolylenediamine, 2-isopropyl-para-phenylenediamine, 2-β-hydroxyethyl-para-phenylenediamine, 2-β-hydroxyethyloxy-para-phenylenediamine, 2,6-dimethyl-para-phenylenediamine, 2,6-diethyl-para-phenylenediamine, 2,3-dimethyl-para-phenylenediamine, N,N-bis(β-hydroxyethyl)-para-phenylenediamine, 2-chloro-para-phenylenediamine and 2-β-acetylaminoethyloxy-para-phenylenediamine, and addition salts thereof with an acid, are particularly preferred.

Among the bis(phenyl)alkylenediamines, examples that may be mentioned include N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)ethylenediamine, 1,8-bis(2,5-diaminophenoxy)-3,6-dioxaoctane, and addition salts thereof.

Among the para-aminophenols, examples that may be mentioned include para-aminophenol, 4-amino-3-methylphenol, 4-amino-3-fluorophenol, 4-amino-3-chlorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

Among the ortho-aminophenols, examples that may be mentioned include 2-aminophenol, 2-amino-5-methylphenol, 2-amino-6-methylphenol and 5-acetamido-2-aminophenol, and addition salts thereof.

Among the heterocyclic bases, examples that may be mentioned include pyridine derivatives, pyrimidine derivatives and pyrazole derivatives.

Among the pyridine derivatives, mention may be made of the compounds described for example in patents GB 1 026 978 and GB 1 153 196, for instance 2,5-diaminopyridine, 2-(4-methoxyphenyl)amino-3-aminopyridine and 3,4-diaminopyridine, and addition salts thereof.

Other pyridine oxidation bases that are useful in the present invention are the 3-aminopyrazolo[1,5-a]pyridine oxidation bases or addition salts thereof described, for example, in patent application FR 2 801 308. Examples that may be mentioned include pyrazolo[1,5-a]pyrid-3-ylamine, 2-acetylaminopyrazolo[1,5-a]pyrid-3-ylamine, 2-morpholin-4-ylpyrazolo[1,5-a]pyrid-3-ylamine, 3-aminopyrazolo[1,5-a]pyridine-2-carboxylic acid, 2-methoxypyrazolo[1,5-a]pyrid-3-ylamine, (3-aminopyrazolo[1,5-a]pyrid-7-yl)methanol, 2-(3-aminopyrazolo[1,5-a]pyrid-5-yl)ethanol, 2-(3-aminopyrazolo[1,5-a]pyrid-7-yl)ethanol, (3-aminopyrazolo[1,5-a]pyrid-2-yl)methanol, 3,6-diaminopyrazolo[1,5-a]pyridine, 3,4-diaminopyrazolo[1,5-a]pyridine, pyrazolo[1,5-a]pyridine-3,7-diamine, 2-(3-aminopyrazolo[1,5-a]pyrid-2-yloxy)ethanol, 7-morpholin-4-ylpyrazolo[1,5-a]pyrid-3-ylamine, pyrazolo[1,5-a]pyridine-3,5-diamine, 5-morpholin-4-ylpyrazolo[1,5-a]pyrid-3-ylamine, 2-[(3-aminopyrazolo[1,5-a]pyrid-5-yl)(2-hydroxyethyl)amino]ethanol, 2-[(3-aminopyrazolo[1,5-a]pyrid-7-yl)(2-hydroxyethyl)amino]ethanol, 3-aminopyrazolo[1,5-a]pyridin-5-ol, 3-aminopyrazolo[1,5-a]pyridin-4-ol, 3-aminopyrazolo[1,5-a]pyridin-6-ol and 3-aminopyrazolo[1,5-a]pyridin-7-ol, and addition salts thereof. Salts of 2-(3-aminopyrazolo[1,5-a]pyrid-2-yloxy)ethanol are particularly appreciated.

Among the pyrimidine derivatives, mention may be made of the compounds described, for example, in patents DE 2359399, JP 88-169571, JP 05-63124 and EP 0770375 or patent application WO 96/15765, such as 2,4,5,6-tetraaminopyrimidine, 4-hydroxy-2,5,6-triaminopyrimidine, 2-hydroxy-4,5,6-triaminopyrimidine, 2,4-dihydroxy-5,6-diaminopyrimidine, 2,5,6-triaminopyrimidine and the addition salts thereof, and the tautomeric forms thereof, when a tautomeric equilibrium exists.

Among the pyrazole derivatives, examples that may be mentioned include 3,4-diaminopyrazole, 4-amino-1,3-dimethyl-5-hydrazinopyrazole, 1-methyl-3,4,5-triaminopyrazole, 3,5-diamino-1-methyl-4-methylaminopyrazole and 3,5-diamino-4-(β-hydroxyethyl)amino-1-methylpyrazole, and the addition salts thereof.

Among the couplers that may be used in the composition according to the invention, mention may be made

especially of meta-phenylenediamines, meta-aminophenols, meta-diphenols, naphthalene-based couplers, heterocyclic couplers, for instance indole derivatives, indoline derivatives, sesamol and derivatives thereof, pyridine derivatives, pyrazolotriazole derivatives, pyrazolones, indazoles, benzimidazoles, benzothiazoles, benzoxazoles, 1,3-benzodioxoles, quinolines, and the addition salts of these compounds with an acid.

These couplers are more particularly chosen from 2,4-diamino-1-(β-hydroxyethyloxy)benzene, 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2-amino-4-(β-hydroxyethylamino)-1-methoxybenzene, 1,3-diaminobenzene, 1,3-bis(2,4-diaminophenoxy)propane, sesamol, 1-amino-2-methoxy-4,5-methylenedioxybenzene, α-naphthol, 6-hydroxyindole, 4-hydroxyindole, 4-hydroxy-N-methylindole, 6-hydroxyindoline, 2,6-dihydroxy-4-methylpyridine, 1-H-3-methylpyrazol-5-one, 1-phenyl-3-methylpyrazol-5-one, 2-amino-3-hydroxypyridine, 3,6-dimethylpyrazolo[3,2-c]-1,2,4-triazole and 2,6-dimethylpyrazolo[1,5-b]-1,2,4-triazole, the addition salts thereof with an acid, and mixtures thereof.

The addition salts of the oxidation bases and of the couplers are in particular chosen from the addition salts with an acid such as the hydrochlorides, hydrobromides, sulfates, citrates, succinates, tartrates, lactates, tosylates, benzenesulfonates, phosphates and acetates.

The oxidation base(s) are each generally present in an amount of from 0.0001% to 10% by weight relative to the total weight of the composition of the invention, and preferably from 0.005% to 5% by weight relative to the total weight of the composition.

The coupler(s) each generally represent from 0.0001% to 10% by weight relative to the total weight of the composition, and preferably from 0.005% to 5% by weight relative to the total weight of the composition of the invention.

The direct dyes may be chosen from ionic or nonionic species, preferably cationic or nonionic species. These direct dyes may be synthetic or of natural origin.

Examples of suitable direct dyes that may be mentioned include azo dyes; methine dyes; carbonyl dyes; azine dyes; nitro(hetero)aryl dyes; tri(hetero)arylmethane dyes; porphyrin dyes; phthalocyanin dyes, and natural direct dyes, alone or as mixtures.

When they are present, the direct dye(s) more particularly represent from 0.0001% to 10% by weight and preferably from 0.005% to 5% by weight relative to the total weight of the composition.

### ***Polyols***

According to an advantageous embodiment, the composition according to the invention comprises at least one saturated or unsaturated, linear or branched $C_2$-$C_8$ and preferably $C_3$-$C_6$ polyol, comprising from 2 to 6 hydroxyl groups. Preferably, the polyol is chosen from glycerol, propylene glycol, 1,3-butylene glycol, dipropylene glycol and diglycerol, and mixtures thereof, and more preferably the polyol is chosen from glycerol and propylene glycol.

Preferably, the total content of polyol(s), when they are present, represents from 0.1% to 25% by weight, preferably from 1% to 20% by weight and more particularly from 2% to 15% by weight, relative to the weight of the composition.

### ***Fatty substances***

The dye composition according to the invention may optionally also comprise one or more fatty substances.

The term "fatty substance" means an organic compound that is insoluble in water at ordinary temperature (25°C) and at atmospheric pressure (760 mmHg) (solubility of less than 5%, preferably less than 1% and even more preferentially less than 0.1%). They bear in their structure at least one hydrocarbon-based chain comprising at least 6 carbon atoms or a sequence of at least two siloxane groups. In addition, the fatty substances are generally soluble in organic solvents under the same temperature and pressure conditions, for instance chloroform, dichloromethane, carbon tetrachloride, ethanol, benzene, toluene, tetrahydrofuran (THF), liquid petroleum jelly or decamethylcyclopentasiloxane.

Preferably, the fatty substances of the invention do not contain any salified or unsalified carboxylic acid groups (-C(O)OH or $-C(O)O^-$). Particularly, the fatty substances of the invention are neither polyoxyalkylenated nor polyglycerolated.

The fatty substance may be chosen in particular from oils and solid fatty substances.

The term "*oil*" means a *"fatty substance"* that is liquid at room temperature (25°C) and at atmospheric pressure (760 mmHg).

The term *"non-silicone fatty substance"* means a fatty substance not containing any silicon atoms (Si) and the term *"silicone fatty substance"* means a fatty substance containing at least one silicon atom.

More particularly, the fatty substances are chosen from $C_6$-$C_{16}$ hydrocarbons, hydrocarbons containing more than 16 carbon atoms, non-silicone oils of animal origin, plant oils of triglyceride type, synthetic triglycerides, fluoro oils, fatty alcohols, esters of fatty acids and/or of fatty alcohols other than triglycerides, and plant waxes, non-silicone waxes and silicones.

It is recalled that, for the purposes of the invention, the fatty alcohols, fatty esters and fatty acids more particularly

contain one or more linear or branched, saturated or unsaturated hydrocarbon-based groups comprising 6 to 30 carbon atoms, which are optionally substituted, in particular with one or more (in particular 1 to 4) hydroxyl groups. If they are unsaturated, these compounds may comprise one to three conjugated or unconjugated carbon-carbon double bonds.

As regards the $C_6$-$C_{16}$ hydrocarbons, they are linear, branched or optionally cyclic, and are preferably alkanes. Examples that may be mentioned include hexane, dodecane and isoparaffins such as isohexadecane and isodecane.

A hydrocarbon-based oil of animal origin that may be mentioned is perhydrosqualene.

The triglyceride oils of plant or synthetic origin are preferably chosen from liquid fatty acid triglycerides containing from 6 to 30 carbon atoms, for instance heptanoic or octanoic acid triglycerides, or alternatively, for example, sunflower oil, corn oil, soybean oil, pumpkin oil, grapeseed oil, sesame oil, hazelnut oil, apricot oil, macadamia oil, arara oil, castor oil, avocado oil, caprylic/capric acid triglycerides, for instance those sold by the company Stéarineries Dubois or those sold under the names Miglyol® 810, 812 and 818 by the company Dynamit Nobel, jojoba oil and shea butter oil.

The linear or branched hydrocarbons of mineral or synthetic origin containing more than 16 carbon atoms are preferably chosen from liquid paraffin or petroleum jelly, petroleum jelly, polydecenes and hydrogenated polyisobutene such as Parleam®.

The fluoro oils may be chosen from perfluoromethylcyclopentane and perfluoro-1,3-dimethylcyclohexane, sold under the names Flutec® PC1 and Flutec® PC3 by the company BNFL Fluorochemicals; perfluoro-1,2-dimethylcyclobutane; perfluoroalkanes such as dodecafluoropentane and tetradecafluorohexane, sold under the names PF 5050® and PF 5060® by the company 3M, or alternatively bromoperfluorooctyl sold under the name Foralkyl® by the company Atochem; nonafluoromethoxybutane and nonafluoroethoxyisobutane; perfluoromorpholine derivatives such as 4-trifluoromethyl perfluoromorpholine sold under the name PF 5052® by the company 3M.

The fatty alcohols that may be used in the composition according to the invention are saturated or unsaturated, and linear or branched, and comprise from 6 to 30 carbon atoms and more particularly from 8 to 18 carbon atoms. Examples that may be mentioned include cetyl alcohol, stearyl alcohol and the mixture thereof (cetylstearyl alcohol), octyldodecanol, 2-butyloctanol, 2-hexyldecanol, 2-undecylpentadecanol, oleyl alcohol and linoleyl alcohol.

The wax(es) that may be used in the composition according to the invention are chosen especially from carnauba wax, candelilla wax, esparto grass wax, paraffin wax, ozokerite, plant waxes, for instance olive tree wax, rice wax, hydrogenated jojoba wax or the absolute waxes of flowers such as the essential wax of blackcurrant blossom sold by the company Bertin (France), animal waxes, for instance beeswaxes, or modified beeswaxes (cerabellina); other waxes or waxy starting materials that may be used according to the invention are especially marine waxes such as the product sold by the company Sophim under the reference M82, and polyethylene waxes or polyolefin waxes in general.

As regards the fatty acid and/or fatty alcohol esters, which are advantageously different from the triglycerides mentioned above, mention may be made in particular of esters of saturated or unsaturated, linear or branched $C_1$-$C_{26}$ aliphatic mono- or polyacids and of saturated or unsaturated, linear or branched $C_1$-$C_{26}$ aliphatic mono- or polyalcohols, the total carbon number of the esters more particularly being greater than or equal to 10.

Among the monoesters, mention may be made of dihydroabietyl behenate; octyldodecyl behenate; isocetyl behenate; cetyl lactate; $C_{12}$-$C_{15}$ alkyl lactate; isostearyl lactate; lauryl lactate; linoleyl lactate; oleyl lactate; (iso)stearyl octanoate; isocetyl octanoate; octyl octanoate; cetyl octanoate; decyl oleate; isocetyl isostearate; isocetyl laurate; isocetyl stearate; isodecyl octanoate; isodecyl oleate; isononyl isononanoate; isostearyl palmitate; methyl acetyl ricinoleate; myristyl stearate; octyl isononanoate; 2-ethylhexyl isononanoate; octyl palmitate; octyl pelargonate; octyl stearate; octyldodecyl erucate; oleyl erucate; ethyl and isopropyl palmitates, 2-ethylhexyl palmitate, 2-octyldecyl palmitate, alkyl myristates such as isopropyl, butyl, cetyl, 2-octyldodecyl, myristyl or stearyl myristate, hexyl stearate, butyl stearate, isobutyl stearate; dioctyl malate, hexyl laurate, 2-hexyldecyl laurate.

Still within the context of this variant, esters of $C_4$-$C_{22}$ dicarboxylic or tricarboxylic acids and of $C_1$-$C_{22}$ alcohols and esters of mono-, di- or tricarboxylic acids and of $C_2$-$C_{26}$ di-, tri-, tetra- or pentahydroxy alcohols may also be used.

Mention may be made especially of: diethyl sebacate; diisopropyl sebacate; diisopropyl adipate; di-n-propyl adipate; dioctyl adipate; diisostearyl adipate; dioctyl maleate; glyceryl undecylenate; octyldodecyl stearoyl stearate; pentaerythrityl monoricinoleate; pentaerythrityl tetraisononanoate; pentaerythrityl tetrapelargonate; pentaerythrityl tetraisostearate; pentaerythrityl tetraoctanoate; propylene glycol dicaprylate; propylene glycol dicaprate; tridecyl erucate; triisopropyl citrate; triisostearyl citrate; glyceryl trilactate; glyceryl trioctanoate; trioctyldodecyl citrate; trioleyl citrate; propylene glycol dioctanoate; neopentyl glycol diheptanoate; diethylene glycol diisononanoate; and polyethylene glycol distearates.

Among the esters mentioned above, it is preferred to use ethyl, isopropyl, myristyl, cetyl or stearyl palmitate, 2-ethylhexyl palmitate, 2-octyldecyl palmitate, alkyl myristates such as isopropyl, butyl, cetyl or 2-octyldodecyl myristate, hexyl stearate, butyl stearate, isobutyl stearate; dioctyl malate, hexyl laurate, 2-hexyldecyl laurate, isononyl isononanoate or cetyl octanoate.

The composition may also comprise, as fatty ester, sugar esters and diesters of $C_6$-$C_{30}$ and preferably $C_{12}$-$C_{22}$ fatty acids. It is recalled that the term "sugar" means oxygen-bearing hydrocarbon-based compounds bearing several alcohol functions, with or without aldehyde or ketone functions, and which comprise at least 4 carbon atoms. These

sugars may be monosaccharides, oligosaccharides or polysaccharides.

Examples of suitable sugars that may be mentioned include sucrose (or saccharose), glucose, galactose, ribose, fucose, maltose, fructose, mannose, arabinose, xylose and lactose, and derivatives thereof, especially alkyl derivatives, such as methyl derivatives, for instance methylglucose.

The sugar esters of fatty acids may be chosen in particular from the group comprising the esters or mixtures of esters of sugars described previously and of linear or branched, saturated or unsaturated $C_6$-$C_{30}$ and preferably $C_{12}$-$C_{22}$ fatty acids. If they are unsaturated, these compounds may comprise one to three conjugated or unconjugated carbon-carbon double bonds.

The esters according to this variant may also be chosen from mono-, di-, tri- and tetraesters, polyesters, and mixtures thereof.

These esters may be, for example, oleates, laurates, palmitates, myristates, behenates, cocoates, stearates, linoleates, linolenates, caprates and arachidonates, or mixtures thereof such as, in particular, oleopalmitate, oleostearate and palmitostearate mixed esters.

More particularly, use is made of monoesters and diesters and in particular sucrose, glucose or methylglucose monooleate or dioleate, stearate, behenate, oleopalmitate, linoleate, linolenate or oleostearate.

An example that may be mentioned is the product sold under the name Glucate® DO by Amerchol, which is a methylglucose dioleate.

Examples of esters or mixtures of esters of sugar and of fatty acid that may also be mentioned include:

- the products sold under the names F160, F140, F110, F90, F70 and SL40 by the company Crodesta, respectively denoting sucrose palmitate/stearates formed from 73% monoester and 27% diester and triester, from 61% monoester and 39% diester, triester and tetraester, from 52% monoester and 48% diester, triester and tetraester, from 45% monoester and 55% diester, triester and tetraester, from 39% monoester and 61% diester, triester and tetraester, and sucrose monolaurate;
- the products sold under the name Ryoto Sugar Esters, for example referenced B370 and corresponding to sucrose behenate formed from 20% monoester and 80% diester-triester-polyester;
- the sucrose mono-dipalmito-stearate sold by the company Goldschmidt under the name Tegosoft® PSE.

The silicones that may be used in accordance with the invention may be in the form of oils, waxes, resins or gums.

Preferably, the silicone is chosen from polydialkylsiloxanes, in particular polydimethylsiloxanes (PDMSs), and organomodified polysiloxanes including at least one functional group chosen from amino groups, aryl groups and alkoxy groups.

Organopolysiloxanes are defined in greater detail in Walter Noll's Chemistry and Technology of Silicones (1968), Academic Press. They may be volatile or non-volatile.

When they are volatile, the silicones are more particularly chosen from those with a boiling point of between 60°C and 260°C, and even more particularly from:

(i) cyclic polydialkylsiloxanes comprising from 3 to 7 and preferably from 4 to 5 silicon atoms. These are, for example, octamethylcyclotetrasiloxane sold in particular under the name Volatile Silicone® 7207 by Union Carbide or Silbione® 70045 V2 by Rhodia, decamethylcyclopentasiloxane sold under the name Volatile Silicone® 7158 by Union Carbide, and Silbione® 70045 V5 by Rhodia, and mixtures thereof.

Mention may also be made of cyclocopolymers of the dimethylsiloxane/methylalkylsiloxane type, such as Volatile Silicone® FZ 3109 sold by Union Carbide, having the formula: with D" :

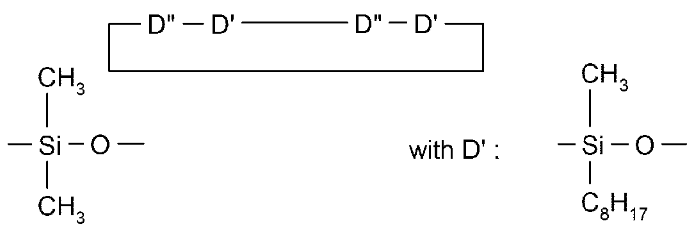

with D' :

Mention may also be made of mixtures of cyclic polydialkylsiloxanes with organosilicon compounds, such as the mixture of octamethylcyclotetrasiloxane and tetra(trimethylsilyl)pentaerythritol (50/50) and the mixture of octamethylcyclotetrasiloxane and oxy-1,1'-bis(2,2,2',2',3,3'-hexatrimethylsilyloxy)neopentane;

(ii) linear volatile polydialkylsiloxanes containing 2 to 9 silicon atoms and having a viscosity of less than or equal to $5\times10^{-6}$ $m^2/s$ at 25°C. An example is decamethyltetrasiloxane sold in particular under the name SH 200 by the

company Toray Silicone. Silicones belonging to this category are also described in the article published in Cosmetics and Toiletries, Vol. 91, Jan. 76, pp. 27-32, Todd & Byers, "Volatile Silicone Fluids for Cosmetics".

Non-volatile polydialkylsiloxanes, polydialkylsiloxane gums and resins, polyorganosiloxanes modified with the above organofunctional groups, and mixtures thereof, are preferably used.

These silicones are more particularly chosen from polydialkylsiloxanes, among which mention may be made mainly of polydimethylsiloxanes bearing trimethylsilyl end groups. The viscosity of the silicones is measured at 25°C according to ASTM Standard 445 Appendix C.

Among these polydialkylsiloxanes, mention may be made, in a non-limiting manner, of the following commercial products:

- the Silbione® oils of the 47 and 70 047 series or the Mirasil® oils sold by Rhodia, for instance the oil 70 047 V 500 000;
- the oils of the Mirasil® series sold by Rhodia;
- the oils of the 200 series from the company Dow Corning, such as DC200 with a viscosity of 60 000 $mm^2/s$;
- the Viscasil® oils from General Electric and certain oils of the SF series (SF 96, SF 18) from General Electric.

Mention may also be made of polydimethylsiloxanes bearing dimethylsilanol end groups, known under the name dimethiconol (CTFA), such as the oils of the 48 series from the company Rhodia.

In this category of polydialkylsiloxanes, mention may also be made of the products sold under the names Abil Wax® 9800 and 9801 by the company Goldschmidt, which are polydi($C_1$-$C_{20}$)alkylsiloxanes.

The silicone gums that may be used in accordance with the invention are especially polydialkylsiloxanes and preferably polydimethylsiloxanes with high number-average molecular masses of between 200 000 and 1 000 000, used alone or as a mixture in a solvent. This solvent may be chosen from volatile silicones, polydimethylsiloxane (PDMS) oils, polyphenylmethylsiloxane (PPMS) oils, isoparaffins, polyisobutylenes, methylene chloride, pentane, dodecane and tridecane, or mixtures thereof.

Products that may be used more particularly in accordance with the invention are mixtures such as:

- mixtures formed from a polydimethylsiloxane with a hydroxy-terminated chain, or dimethiconol (CTFA), and from a cyclic polydimethylsiloxane, also known as cyclomethicone (CTFA), such as the product Q2 1401 sold by the company Dow Corning;
- mixtures of a polydimethylsiloxane gum and a cyclic silicone, such as the product SF 1214 Silicone Fluid from the company General Electric; this product is an SF 30 gum corresponding to a dimethicone, having a number-average molecular weight of 500 000, dissolved in the oil SF 1202 Silicone Fluid corresponding to decamethylcyclopentasiloxane;
- mixtures of two PDMSs with different viscosities, and more particularly of a PDMS gum and a PDMS oil, such as the product SF 1236 from the company General Electric. The product SF 1236 is the mixture of a gum SE 30 defined above, with a viscosity of 20 $m^2/s$ and of an oil SF 96 with a viscosity of $5\times10^{-6}$ $m^2/s$. This product preferably comprises 15% of gum SE 30 and 85% of an oil SF 96.

The organopolysiloxane resins that may be used in accordance with the invention are crosslinked siloxane systems containing the following units:

$$R_2SiO_{2/2},\ R_3SiO_{1/2},\ RSiO_{3/2} \text{ and } SiO_{4/2},$$

in which R represents an alkyl containing 1 to 16 carbon atoms. Among these products, the ones that are particularly preferred are those in which R denotes a $C_1$-$C_4$ lower alkyl group, more particularly methyl.

Among these resins, mention may be made of the product sold under the name Dow Corning 593 or those sold under the names Silicone Fluid SS 4230 and SS 4267 by General Electric, which are silicones of dimethyl/trimethylsiloxane structure.

Mention may also be made of the trimethyl siloxysilicate-type resins sold especially under the names X22-4914, X21-5034 and X21-5037 by Shin-Etsu.

The organomodified silicones that may be used in accordance with the invention are silicones as defined above and comprising in their structure one or more organofunctional groups attached via a hydrocarbon-based group.

The organomodified silicones may be polydiarylsiloxanes, especially polydiphenylsiloxanes, and polyalkylarylsiloxanes functionalized with the organofunctional groups mentioned previously.

The polyalkylarylsiloxanes are particularly chosen from linear and/or branched polydimethyl/methylphenylsiloxanes and polydimethyl/diphenylsiloxanes with a viscosity ranging from $1\times10^{-5}$ to $5\times10^{-2}$ $m^2/s$ at 25°C.

Among these polyalkylarylsiloxanes, examples that may be mentioned include the products sold under the

following names:

- the Silbione® oils of the 70 641 series from Rhodia;
- the oils of the Rhodorsil® 70 633 and 763 series from Rhodia;
- the oil Dow Corning 556 Cosmetic Grade Fluid from Dow Corning;
- the silicones of the PK series from Bayer, such as the product PK20;
- the silicones of the PN and PH series from Bayer, such as the products PN1000 and PH1000;
- certain oils of the SF series from General Electric, such as SF 1023, SF 1154, SF 1250 and SF 1265.

Among the organomodified silicones, mention may also be made of polyorganosiloxanes comprising:

- substituted or unsubstituted amino groups, such as the products sold under the names GP 4 Silicone Fluid and GP 7100 by the company Genesee or the products sold under the names Q2 8220 and Dow Corning 929 or 939 by the company Dow Corning. The substituted amino groups are, in particular, $C_1$-$C_4$ aminoalkyl groups;
- alkoxy groups such as the product sold under the name Silicone Copolymer F-755 by SWS Silicones, and Abil Wax® 2428, 2434 and 2440 by the company Goldschmidt.

Preferably, the fatty substances that may be used in the composition according to the invention are non-silicone fatty substances.

The fatty substances are advantageously chosen from $C_6$-$C_{16}$ hydrocarbons, hydrocarbons containing more than 16 carbon atoms, triglycerides, fatty alcohols, esters of fatty acids and/or of fatty alcohols other than triglycerides, or mixtures thereof.

Preferably, the fatty substance(s) are chosen from liquid petroleum jelly, liquid paraffin, polydecenes, fatty alcohols and esters of fatty acids and/or of fatty alcohols, or mixtures thereof.

Even more preferentially, the fatty substances are chosen from liquid petroleum jelly, liquid paraffin and fatty alcohols, and mixtures thereof.

According to one embodiment, the composition according to the invention comprises at least one oil, preferably liquid petroleum jelly or liquid paraffin, and at least one fatty alcohol, preferably chosen from fatty alcohols comprising from 6 to 30 carbon atoms, as described above, in particular chosen from cetyl alcohol, stearyl alcohol and a mixture thereof (cetylstearyl alcohol).

The fatty substance(s) may be present in a content ranging from 1% to 20% by weight, more preferentially from 2% to 15% by weight and preferably from 5% to 12% by weight relative to the weight of the composition.

According to one embodiment, the composition according to the invention comprises less than 20% by weight of fatty substances, preferably less than 15% of fatty substances, relative to the total weight of the composition of the invention.

According to an advantageous embodiment, the fatty substance content in the composition is greater than or equal to 1% by weight, preferably greater than or equal to 2% by weight, better still greater than or equal to 5% by weight and even better still greater than or equal to 7% by weight relative to the total weight of the composition.

*c)* ***Surfactants***

The dye composition also comprises one or more surfactants.

According to a particular embodiment of the invention, the surfactant(s) are chosen from anionic, cationic, nonionic and amphoteric surfactants, and preferentially nonionic surfactants.

The term *"anionic surfactant"* means a surfactant comprising, as ionic or ionizable groups, only anionic groups. These anionic groups are preferably chosen from the following groups: -C(O)-OH, $-C(O)-O^-$, $-SO_3H$, $-S(O)_2O^-$, $-OS(O)_2OH$, $-OS(O)_2O^-$, $-P(O)OH_2$, $-P(O)_2O^-$, $-P(O)O_2^-$, $-P(OH)_2$, =P(O)OH, $-P(OH)O^-$, $=P(O)O^-$, =POH, $=PO^-$; the anionic parts comprising a cationic counterion such as an alkali metal, an alkaline-earth metal or an ammonium.

As examples of anionic surfactants that may be used in the composition according to the invention, mention may be made of alkyl sulfates, alkyl ether sulfates, alkylamido ether sulfates, alkylaryl polyether sulfates, monoglyceride sulfates, alkylsulfonates, alkylamidesulfonates, alkylarylsulfonates, $\alpha$-olefin sulfonates, paraffin sulfonates, alkyl sulfosuccinates, alkyl ether sulfosuccinates, alkylamide sulfosuccinates, alkyl sulfoacetates, acylsarcosinates, acylglutamates, alkyl sulfosuccinamates, acylisethionates and N-acyltaurates, polyglycoside polycarboxylic acid and alkyl monoester salts, acyl lactylates, alkyl or alkenyl phosphates, salts of D-galactoside uronic acids, salts of alkyl ether carboxylic acids, salts of alkylaryl ether carboxylic acids, salts of alkylamido ether carboxylic acids; and the corresponding non-salified forms of all these compounds; the alkyl and acyl groups of all these compounds comprising from 6 to 24 carbon atoms and the aryl group denoting a phenyl group.

These compounds may be oxyethylenated and then preferably comprise from 1 to 50 ethylene oxide units.

The salts of $C_6$-$C_{24}$ alkyl monoesters of polyglycoside-polycarboxylic acids may be chosen from $C_6$-$C_{24}$ alkyl polyglycoside-citrates, $C_6$-$C_{24}$ alkyl polyglycoside-tartrates and $C_6$-$C_{24}$ alkyl polyglycoside-sulfosuccinates.

When the anionic surfactant(s) are in salt form, they may be chosen from alkali metal salts such as the sodium or potassium salt and preferably the sodium salt, ammonium salts, amine salts and in particular amino alcohol salts or alkaline-earth metal salts such as the magnesium salts.

Examples of amino alcohol salts that may especially be mentioned include monoethanolamine, diethanolamine and triethanolamine salts, monoisopropanolamine, diisopropanolamine and triisopropanolamine salts, 2-amino-2-methyl-1-propanol salts, 2-amino-2-methyl-1,3-propanediol salts and tris(hydroxymethyl)aminomethane salts.

Use is preferably made of alkali metal or alkaline-earth metal salts, and in particular sodium or magnesium salts.

Among the anionic surfactants mentioned, use is preferably made of ($C_6$-$C_{24}$)alkyl sulfates, ($C_6$-$C_{24}$)alkyl ether sulfates comprising from 2 to 50 ethylene oxide units, in particular in the form of alkali metal, ammonium, amino alcohol and alkaline-earth metal salts, or a mixture of these compounds.

It is in particular preferred to use ($C_{12}$-$C_{20}$)alkyl sulfates, ($C_{12}$-$C_{20}$)alkyl ether sulfates comprising from 2 to 20 ethylene oxide units, in particular in the form of alkali metal, ammonium, amino alcohol and alkaline-earth metal salts, or a mixture of these compounds. Better still, it is preferred to use sodium lauryl ether sulfate containing 2.2 mol of ethylene oxide.

According to one embodiment, the composition according to the invention comprises at least one additional surfactant chosen from anionic surfactants, in particular from ($C_6$-$C_{24}$)alkyl sulfates.

The cationic surfactant(s) that may be used in the composition according to the invention comprise, for example, optionally polyoxyalkylenated primary, secondary or tertiary fatty amine salts, quaternary ammonium salts, and mixtures thereof.

Examples of quaternary ammonium salts that may especially be mentioned include:

- those corresponding to the general formula (A4) below:

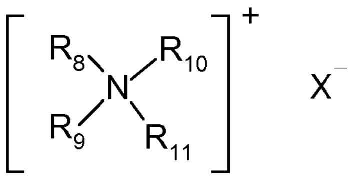

(A4)

in which formula (A4):

  - $R_8$ to $R_{11}$, which may be identical or different, represent a linear or branched aliphatic group comprising from 1 to 30 carbon atoms, or an aromatic group such as aryl or alkylaryl, it being understood that at least one of the groups $R_8$ to $R_{11}$ comprises from 8 to 30 carbon atoms and preferably from 12 to 24 carbon atoms; and

  - $X^-$ represents an organic or inorganic anionic counterion, such as that chosen from halides, acetates, phosphates, nitrates, ($C_1$-$C_4$)alkyl sulfates, ($C_1$-$C_4$)alkyl or ($C_1$-$C_4$)alkylaryl sulfonates, in particular methyl sulfate and ethyl sulfate.

The aliphatic groups of $R_8$ to $R_{11}$ may also comprise heteroatoms in particular such as oxygen, nitrogen, sulfur and halogens.

The aliphatic groups of $R_8$ to $R_{11}$ are chosen, for example, from $C_1$-$C_{30}$ alkyl, $C_1$-$C_{30}$ alkoxy, polyoxy(C2-C6)alkylene, $C_1$-$C_{30}$ alkylamide, ($C_{12}$-$C_{22}$)alkylamido($C_2$-$C_6$)alkyl, ($C_{12}$-$C_{22}$)alkyl acetate, and $C_1$-$C_{30}$ hydroxyalkyl groups; $X^-$ is an anionic counterion chosen from halides, phosphates, acetates, lactates, ($C_1$-$C_4$)alkyl sulfates, and ($C_1$-$C_4$)alkyl or ($C_1$-$C_4$)alkylaryl sulfonates.

Among the quaternary ammonium salts of formula (A4), preference is given firstly to tetraalkylammonium chlorides, for instance dialkyldimethylammonium or alkyltrimethylammonium chlorides in which the alkyl group contains approximately from 12 to 22 carbon atoms, in particular behenyltrimethylammonium chloride, distearyldimethylammonium chloride, cetyltrimethylammonium chloride, benzyldimethylstearylammonium chloride, or else, secondly, distearoylethylhydroxyethylmethylammonium methosulfate, dipalmitoylethylhydroxyethylammonium methosulfate or distearoylethylhydroxyethylammonium methosulfate, or else, lastly, palmitylamidopropyltrimethylammonium chloride or stearamidopropyldimethyl(myristyl acetate)ammonium chloride, sold under the name Ceraphyl® 70 by the company

Van Dyk;

- quaternary ammonium salts of imidazoline, for instance those of formula (A5) below:

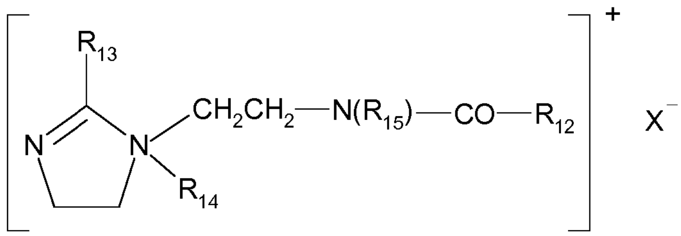

(A5)

in which formula (A5):

- $R_{12}$ represents an alkenyl or alkyl group comprising from 8 to 30 carbon atoms, for example derived from fatty acids of tallow;

- $R_{13}$ represents a hydrogen atom, a $C_1$-$C_4$ alkyl group or an alkenyl or alkyl group comprising from 8 to 30 carbon atoms;

- $R_{14}$ represents a $C_1$-$C_4$ alkyl group;

- $R_{15}$ represents a hydrogen atom or a $C_1$-$C_4$ alkyl group;

- $X^-$ represents an organic or inorganic anionic counterion, such as that chosen from halides, phosphates, acetates, lactates, ($C_1$-$C_4$)alkyl sulfates, ($C_1$-$C_4$)alkyl or ($C_1$-$C_4$)alkylaryl sulfonates.

Preferably, $R_{12}$ and $R_{13}$ denote a mixture of alkenyl or alkyl groups comprising from 12 to 21 carbon atoms, for example derived from tallow fatty acids, $R_{14}$ denotes a methyl group and $R_{15}$ denotes a hydrogen atom. Such a product is sold, for example, under the name Rewoquat® W 75 by the company Rewo;

- quaternary diammonium or triammonium salts, particularly of formula (A6) below:

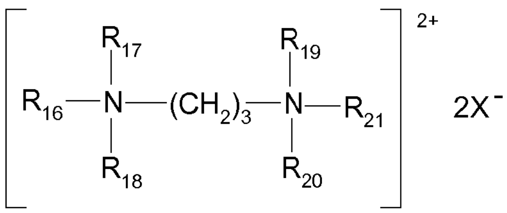

(A6)

in which formula (A6):

- $R_{16}$ denotes an alkyl group comprising approximately from 16 to 30 carbon atoms, which is optionally hydroxylated and/or interrupted with one or more oxygen atoms;
- $R_{17}$ is chosen from hydrogen, an alkyl group comprising from 1 to 4 carbon atoms or a group $-(CH_2)_3-N^+(R_{16a})(R_{17a})(R_{18a})$, $X^-$;
- $R_{16a}$, $R_{17a}$, $R_{18a}$, $R_{18}$, $R_{19}$, $R_{20}$ and $R_{21}$, which may be identical or different, are chosen from hydrogen and an alkyl group comprising from 1 to 4 carbon atoms; and
- $X^-$, which may be identical or different, represent an organic or inorganic anionic counterion, such as that chosen from halides, acetates, phosphates, nitrates, alkyl($C_1$-$C_4$) sulfates, alkyl($C_1$-$C_4$)- or alkyl($C_1$-$C_4$)arylsulfonates, more particularly methyl sulfate and ethyl sulfate.

Such compounds are, for example, Finquat CT-P, made available by the company Finetex (Quaternium 89), and Finquat CT, made available by the company Finetex (Quaternium 75);

- quaternary ammonium salts containing one or more ester functions, such as those of formula (A7) below:

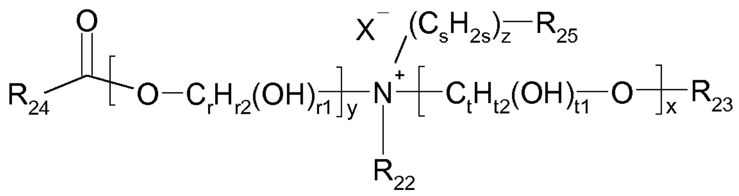

(A7)

in which formula (A7):

- $R_{22}$ is chosen from $C_1$-$C_6$ alkyl groups and $C_1$-$C_6$ hydroxyalkyl or $C_1$-$C_6$ dihydroxyalkyl groups;
- $R_{23}$ is chosen from:

- the group

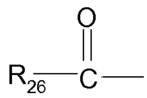
,

- saturated or unsaturated, linear or branched $C_1$-$C_{22}$ hydrocarbon-based groups $R_{27}$,
- a hydrogen atom,

- $R_{25}$ is chosen from:

- the group

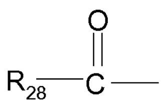
,

- saturated or unsaturated, linear or branched $C_1$-$C_6$ hydrocarbon-based groups $R_{29}$,
- a hydrogen atom,

- $R_{24}$, $R_{26}$ and $R_{28}$, which may be identical or different, are chosen from linear or branched, saturated or unsaturated $C_7$-$C_{21}$ hydrocarbon-based groups;
- r, s and t, which may be identical or different, are integers ranging from 2 to 6,
- r1 and t1, which may be identical or different, are equal to 0 or 1, with r2+r1=2r and t1 +t2=2t,
- y is an integer ranging from 1 to 10,
- x and z, which may be identical or different, are integers ranging from 0 to 10,
- $X^-$ represents an organic or inorganic anionic counterion,

with the proviso that the sum x + y + z is from 1 to 15, that when x is 0 then $R_{23}$ denotes $R_{27}$, and that when z is 0 then $R_{25}$ denotes $R_{29}$.

The alkyl groups $R_{22}$ may be linear or branched, and more particularly linear.

Preferably, $R_{22}$ denotes a methyl, ethyl, hydroxyethyl or dihydroxypropyl group, and more particularly a methyl or ethyl group.

Advantageously, the sum x + y + z ranges from 1 to 10.

When $R_{23}$ is a hydrocarbon-based group $R_{27}$, it may be long and contain from 12 to 22 carbon atoms, or may be short and contain from 1 to 3 carbon atoms.

When $R_{25}$ is a hydrocarbon-based group $R_{29}$, it preferably contains 1 to 3 carbon atoms.

Advantageously, $R_{24}$, $R_{26}$ and $R_{28}$, which may be identical or different, are chosen from linear or branched, saturated or unsaturated $C_{11}$-$C_{21}$ hydrocarbon-based groups, and more particularly from linear or branched, saturated or unsaturated $C_{11}$-$C_{21}$ alkyl and alkenyl groups.

Preferably, x and z, which may be identical or different, are equal to 0 or 1.

Advantageously, y is equal to 1.

Preferably, r, s and t, which may be identical or different, are equal to 2 or 3, and even more particularly are equal to 2.

The anionic counterion $X^-$ is preferably a halide, such as chloride, bromide or iodide; a $(C_1\text{-}C_4)$alkyl sulfate or a $(C_1\text{-}C_4)$alkyl- or $(C_1\text{-}C_4)$alkylarylsulfonate. However, use may be made of methanesulfonate, phosphate, nitrate, tosylate, an anion derived from an organic acid, such as acetate or lactate, or any other anion that is compatible with the ammonium bearing an ester function.

The anionic counterion $X^-$ is even more particularly chloride, methyl sulfate or ethyl sulfate.

Use is made more particularly, in the composition according to the invention, of the ammonium salts of formula (A7) in which:

- $R_{22}$ denotes a methyl or ethyl group,
- x and y are equal to 1,
- z is equal to 0 or 1,
- r, s and t are equal to 2,
- $R_{23}$ is chosen from:
  - the group

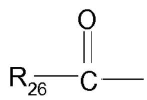

  - methyl, ethyl or $C_{14}\text{-}C_{22}$ hydrocarbon-based groups,
  - a hydrogen atom,

- $R_{25}$ is chosen from:
  - the group

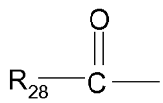

  - a hydrogen atom,

- $R_{24}$, $R_{26}$ and $R_{28}$, which may be identical or different, are chosen from linear or branched, saturated or unsaturated $C_{13}\text{-}C_{17}$ hydrocarbon-based groups, and preferably from linear or branched, saturated or unsaturated $C_{13}\text{-}C_{17}$ alkyl and alkenyl groups.

Advantageously, the hydrocarbon-based radicals are linear.

Among the compounds of formula (A7), examples that may be mentioned include salts, especially the chloride or methyl sulfate, of diacyloxyethyldimethylammonium, diacyloxyethylhydroxyethylmethylammonium, monoacyloxyethyldihydroxyethylmethylammonium, triacyloxyethylmethylammonium or monoacyloxyethylhydroxyethyldimethylammonium, and mixtures thereof. The acyl groups preferably contain 14 to 18 carbon atoms and are obtained more particularly from a plant oil such as palm oil or sunflower oil. When the compound contains several acyl groups, these groups may be identical or different.

These products are obtained, for example, by direct esterification of triethanolamine, triisopropanolamine, alkyldiethanolamine or alkyldiisopropanolamine, which are optionally oxyalkylenated, with fatty acids or with fatty acid mixtures of plant or animal origin, or by transesterification of the methyl esters thereof. This esterification is followed by a quaternization by means of an alkylating agent such as an alkyl halide, preferably methyl or ethyl halide, a dialkyl sulfate, preferably dimethyl or diethyl sulfate, methyl methanesulfonate, methyl para-toluenesulfonate, glycol chlorohydrin or glycerol chlorohydrin.

Such compounds are sold, for example, under the names Dehyquart® by the company Henkel, Stepanquat® by the company Stepan, Noxamium® by the company Ceca or Rewoquat® WE 18 by the company Rewo-Witco.

The composition according to the invention may contain, for example, a mixture of quaternary ammonium

monoester, diester and triester salts with a weight majority of diester salts.

Use may also be made of the ammonium salts containing at least one ester functional group that are described in patents US-A-4 874 554 and US-A-4 137 180.

Use may be made of behenoylhydroxypropyltrimethylammonium chloride made available by Kao under the name Quatarmin BTC 131.

Preferably, the ammonium salts comprising at least one ester function comprise two ester functions.

Among the cationic surfactants that may be present in the composition according to the invention, it is more particularly preferred to choose cetyltrimethylammonium, behenyltrimethylammonium and dipalmitoylethylhydroxyethylmethylammonium salts, and mixtures thereof, and more particularly behenyltrimethylammonium chloride, cetyltrimethylammonium chloride, and dipalmitoylethylhydroxyethylammonium methosulfate, and mixtures thereof.

Additional amphoteric surfactants that may especially be mentioned include betaines and in particular ($C_8$-$C_{20}$)alkylbetaines such as cocoyl betaine, sulfobetaines, ($C_8$-$C_{20}$)alkylsulfobetaines, ($C_8$-$C_{20}$)alkylamido($C_1$-$C_6$)alkylbetaines, such as cocamidopropylbetaine, and ($C_8$-$C_{20}$)alkylamido($C_1$-$C_6$)alkylsulfobetaines.

Examples of nonionic surfactants that may be used in the composition used according to the invention are described, for example, in the 'Handbook of Surfactants' by M.R. Porter, published by Blackie & Son (Glasgow and London), 1991, pages 116-178. They are especially chosen from alcohols, $\alpha$-diols and ($C_1$-$C_{20}$)alkylphenols, these compounds being polyethoxylated, polypropoxylated and/or polyglycerolated, and containing at least one fatty chain comprising, for example, from 8 to 18 carbon atoms, it being possible for the number of ethylene oxide and/or propylene oxide groups to especially range from 1 to 100, and for the number of glycerol groups to especially range from 2 to 30.

Mention may also be made of copolymers of ethylene oxide and of propylene oxide, optionally oxyethylenated fatty acid esters of sorbitan, fatty acid esters of sucrose, polyoxyalkylenated fatty acid esters, optionally oxyalkylenated alkylpolyglycosides, alkylglucoside esters, N-alkylgluctamine and N-acylmethylglucamine derivatives, aldobionamides and amine oxides.

The nonionic surfactants are chosen more particularly from mono- or polyoxyalkylenated and mono- or polyglycerolated nonionic surfactants. The oxyalkylene units are more particularly oxyethylene or oxypropylene units, or a combination thereof, preferably oxyethylene units.

Mention may be made, as examples of oxyalkylenated nonionic surfactants, of:

- oxyalkylenated ($C_8$-$C_{24}$)alkylphenols;
- saturated or unsaturated, linear or branched, oxyalkylenated $C_8$-$C_{30}$ alcohols;
- saturated or unsaturated, linear or branched, oxyalkylenated $C_8$-$C_{30}$ amides;
- esters of saturated or unsaturated, linear or branched, $C_8$-$C_{30}$ acids and of polyethylene glycols;
- polyoxyethylenated esters of saturated or unsaturated, linear or branched, $C_8$-$C_{30}$ acids and of sorbitol;
- saturated or unsaturated oxyethylenated plant oils;
- condensates of ethylene oxide and/or of propylene oxide, *inter alia,* alone or as mixtures;
- oxyethylenated and/or oxypropylenated silicones.

These oxyalkylenated nonionic surfactants may have a number of moles of ethylene oxide ranging from 1 to 100, preferably from 2 to 50 and preferably from 2 to 33.

Advantageously, the nonionic surfactants do not comprise any oxypropylene units.

Preferably, the compositions of the invention comprise at least one anionic or nonionic surfactant, which is preferably oxyethylenated.

In accordance with a preferred embodiment of the invention, the oxyalkylenated nonionic surfactants are chosen from oxyethylenated $C_8$-$C_{30}$ alcohols comprising from 1 to 100 mol of ethylene oxide; polyoxyethylenated esters of linear or branched, saturated or unsaturated $C_8$-$C_{30}$ acids and of sorbitol comprising from 1 to 100 mol of ethylene oxide.

According to one embodiment, the composition according to the invention comprises at least one oxyethylenated nonionic surfactant comprising from 2 to 33 OE units, better still from 10 to 33 OE units.

These oxyethylenated nonionic surfactants are preferably chosen from oxyethylenated derivatives of saturated or unsaturated, linear or branched, preferably linear, $C_8$-$C_{30}$ and preferably $C_{12}$-$C_{22}$ fatty alcohols, for instance cetyl alcohol, oleyl alcohol, oleocetyl alcohol, lauryl alcohol, behenyl alcohol, cetearyl alcohol, stearyl alcohol and isostearyl alcohol, and mixtures thereof.

As oxyethylenated nonionic surfactant comprising less than 10 to 50 OE units, use is preferably made of products of addition of ethylene oxide and lauryl alcohol, for instance lauryl alcohol 2 OE (CTFA name: laureth-2), products of addition of ethylene oxide and stearyl alcohol, for instance stearyl alcohol 2 OE (CTFA name: steareth-2), stearyl alcohol 20 OE (CTFA name: steareth-20), products of addition of ethylene oxide and decyl alcohol, for instance decyl alcohol 3 OE (CTFA name: deceth-3), decyl alcohol 5 OE (CTFA name: deceth-5), products of addition of ethylene oxide and oleocetyl alcohol, for instance oleocetyl alcohol 5 OE (CTFA name: oleoceteth-5), and mixtures thereof.

According to one embodiment, the composition according to the invention comprises at least one oxyethylenated nonionic

surfactant comprising from 2 to 33 OE units, preferably from 10 to 33 OE units, and at least one oxyethylenated nonionic surfactant comprising less than 10 OE units, these surfactants preferably being chosen from oxyethylenated derivatives of $C_8$-$C_{30}$ fatty alcohols, as described above.

The content of surfactants, preferably nonionic surfactants, more particularly ranges from 0.1% to 20% by weight, preferably from 0.5% to 15% by weight and better still from 1% to 10% by weight relative to the weight of the composition.

The composition may especially comprise one or more mineral thickeners chosen from organophilic clays and fumed silicas, or mixtures thereof.

The organophilic clay may be chosen from montmorillonite, bentonite, hectorite, attapulgite and sepiolite, and mixtures thereof. The clay is preferably a bentonite or a hectorite.

These clays may be modified with a chemical compound chosen from quaternary amines, tertiary amines, amine acetates, imidazolines, amine soaps, fatty sulfates, alkylarylsulfonates and amine oxides, and mixtures thereof.

Mention may be made, as organophilic clays, of quaternium-18 bentonites, such as those sold under the names Bentone 3, Bentone 38 and Bentone 38V by Rheox, Tixogel VP by United Catalyst and Claytone 34, Claytone 40 and Claytone XL by Southern Clay; stearalkonium bentonites, such as those sold under the names Bentone 27 by Rheox, Tixogel LG by United Catalyst and Claytone AF and Claytone APA by Southern Clay; and quaternium-18/benzalkonium bentonites, such as those sold under the names Claytone HT and Claytone PS by Southern Clay.

The fumed silicas may be obtained by high-temperature hydrolysis of a volatile silicon compound in an oxyhydrogen flame, producing a finely divided silica. This process makes it possible in particular to obtain hydrophilic silicas bearing a large number of silanol groups at their surface. Such hydrophilic silicas are sold, for example, under the names Aerosil 130®, Aerosil 200®, Aerosil 255®, Aerosil 300® and Aerosil 380® by Degussa and Cab-O-Sil HS-5®, Cab-O-Sil EH-5®, Cab-O-Sil LM-130®, Cab-O-Sil MS-55® and Cab-O-Sil M-5® by Cabot.

It is possible to chemically modify the surface of the silica via chemical reaction in order to reduce the number of silanol groups. It is especially possible to substitute silanol groups with hydrophobic groups: a hydrophobic silica is then obtained.

The hydrophobic groups may be:

- trimethylsiloxyl groups, which are obtained in particular by treating fumed silica in the presence of hexamethyldisilazane. Silicas thus treated are known as Silica silylate according to the CTFA (6th Edition, 1995). They are sold, for example, under the references Aerosil R812® by Degussa and Cab-O-Sil TS-530® by Cabot.
- dimethylsilyloxyl or polydimethylsiloxane groups, which are obtained in particular by treating fumed silica in the presence of polydimethylsiloxane or dimethyldichlorosilane. Silicas thus treated are known as Silica dimethyl silylate according to the CTFA (6th Edition, 1995). They are sold, for example, under the references Aerosil R972® and Aerosil R974® by the company Degussa and Cab-O-Sil TS-610® and Cab-O-Sil TS-720® by the company Cabot.

The fumed silica preferably has a particle size that may be nanometric to micrometric, for example ranging from about 5 to 200 nm.

When it is present, the mineral thickener represents from 1% to 30% by weight relative to the weight of the composition.

The composition may also comprise one or more polymeric organic thickeners.

These polymeric thickeners may be chosen from fatty acid amides (coconut monoethanolamide or diethanolamide, oxyethylenated carboxylic acid monoethanolamide alkyl ether), polymeric thickeners such as cellulose-based thickeners (hydroxyethylcellulose, hydroxypropylcellulose or carboxymethylcellulose), guar gum and derivatives thereof (hydroxypropyl guar), gums of microbial origin (xanthan gum, scleroglucan gum), acrylic acid or acrylamidopropanesulfonic acid crosslinked homopolymers and associative polymers (polymers comprising hydrophilic regions and fatty-chain hydrophobic regions (alkyl or alkenyl containing at least 10 carbon atoms) that are capable, in an aqueous medium, of reversibly combining with each other or with other molecules).

According to a particular embodiment, the organic thickener is chosen from cellulose-based thickeners (hydroxyethylcellulose, hydroxypropylcellulose or carboxymethylcellulose), guar gum and derivatives thereof (hydroxypropyl guar), gums of microbial origin (xanthan gum or scleroglucan gum) and crosslinked acrylic acid or acrylamidopropanesulfonic acid homopolymers, and preferably from cellulose-based thickeners in particular with hydroxyethylcellulose.

The content of organic thickener(s), if they are present, usually ranges from 0.01% to 20% by weight and preferably from 0.1% to 5% by weight relative to the weight of the composition.

The composition according to the invention may also contain various adjuvants conventionally used in compositions for dyeing the hair, such as anionic, nonionic, amphoteric or zwitterionic non-thickening polymers or mixtures thereof; antioxidants; penetrants; sequestrants; fragrances; dispersants; film-forming agents; ceramides; preserving agents; opacifiers.

The above adjuvants are generally present in an amount for each of them of between 0.01% and 20% by weight

relative to the weight of composition.

The composition of the invention may be in various forms, for instance a solution, an emulsion (milk or cream) or a gel, preferably in the form of an emulsion and particularly of a direct emulsion.

The composition according to the invention is preferably an aqueous composition.

The term *"aqueous composition"* means a composition comprising at least 5% water. Preferably, an aqueous composition comprises more than 10% by weight of water and even more advantageously more than 20% by weight of water.

*Solvent*

The composition according to the invention may also comprise one or more water-soluble organic solvents, different from the polyols described previously.

The term "water-soluble compound" means a compound whose solubility in water is greater than or equal to 5% by weight at ordinary temperature (25°C) and at atmospheric pressure (760 mmHg).

Examples of water-soluble organic solvents other than polyols that may be mentioned include linear or branched $C_2$-$C_4$ alkanols, such as ethanol and isopropanol; polyol ethers, for instance 2-butoxyethanol, propylene glycol monomethyl ether, diethylene glycol monomethyl ether and monoethyl ether, and also aromatic alcohols or ethers, for instance benzyl alcohol or phenoxyethanol, and mixtures thereof.

The water-soluble organic solvent(s) other than polyols, if they are present, represent a content usually ranging from 1% to 40% by weight and preferably from 3% to 30% by weight relative to the weight of the composition.
The composition according to the invention is preferably aqueous. The water content may range from 10% to 90% by weight, preferably from 20% to 80% by weight and better still from 30% to 70% by weight relative to the total weight of the composition.
The pH of the compositions according to the invention generally ranges from 7 to 14, preferably from 8 to 13 and better still from 9 to 12.

*Processes of the invention*

Another subject of the invention is a process for dyeing and/or lightening human keratin fibres, in particular the hair, comprising the application, to the keratin fibres, of the composition according to the invention, alone or in the presence of a chemical oxidizing agent, as described above.

When the process uses a chemical oxidizing agent, the latter may be provided by an oxidizing composition.

The oxidizing composition is preferably aqueous.

The dye composition (composition A) and the oxidizing composition (composition B) are preferably mixed in a weight ratio (A)/(B) ranging from 0.2 to 10 and preferably ranging from 0.5 to 2.

At the time of use, the dyeing and/or lightening composition is mixed with an oxidizing composition as described above. The mixture obtained is then applied to the keratin fibres and left on for 3 to 50 minutes approximately, preferably 5 to 30 minutes approximately, and this may then be followed by a step of rinsing and shampooing or of haircare, then rinsing again, and finally drying. The dyeing and/or lightening composition and the oxidizing composition described may be applied sequentially, in any order, with or without intermediate rinsing.

The keratin fibres may be dried at a temperature ranging from 50 to 80°C or left to dry naturally.

The keratin fibres may be dried by means of a hairdryer, a drying hood or a straightening iron so as to perform a hair shaping step.

*Multi-compartment device:*

Another subject of the invention is a device comprising at least two compartments, for dyeing keratin fibres. A first compartment contains the dyeing and/or lightening composition (A) according to the invention and a second compartment contains the oxidizing composition (B) as described above.

The examples that follow illustrate the invention.

**Example 1**

The following compositions were prepared (amounts expressed in grams, unless otherwise mentioned):

| INCI NAME | A1 g% | A2 g% |
|---|---|---|
| Guanidine carbonate | 4 | 4 |

(continued)

| INCI NAME | A1 g% | A2 g% |
|---|---|---|
| Liquid petroleum jelly | 11.6 | 11.6 |
| Cetearyl alcohol | 6 | 6 |
| Camellia oil | 0.2 | 0.2 |
| | | |
| Hexadimethrine chloride (Mexomer PO from Chimex) | 0.3 AM | |
| Polyquaternium-6 (Merquat 100 from Lubrizol) | 0.4 AM | 0.7 AM |
| Steareth-2 | 1.38 | 1.38 |
| Steareth-20 | 2.75 | 2.75 |
| Ammonium hydroxide | 2.47 | 2.47 |
| Monoethanolamine | 2 | 2 |
| Sodium metabisulfite | 0.7 | 0.7 |
| Erythorbic acid | 0.3 | 0.3 |
| Propylene glycol | 10 | 10 |
| Glycerol | 3 | 3 |
| EDTA | 0.2 | 0.2 |
| Fragrance | qs | qs |
| Water | qs 100 | qs 100 |
| AM: active material | | |

**Oxidizing composition**

| | g% |
|---|---|
| 50% Aqueous hydrogen peroxide solution | 6 AM |
| Tetrasodium pyrophosphate | 0.02 |
| Pentasodium pentetate | 0.15 |
| Glycerol | 0.5 |
| Cetylstearyl alcohol/oxyethylenated (30 OE) cetylstearyl alcohol mixture (Nonidac 1618 F from Sasol) | 2.85 |
| Trideceth-2 carboxamide MEA (Amidet A15/LAO 55 from Kao) | 0.85 |
| Phosphoric acid | qs pH 2.2 |
| Sodium stannate | 0.04 |
| Water | qs 100 |

At the time of use, 1 part by weight of composition A1 or A2 is mixed with 1.5 parts by weight of composition B.

Each mixture obtained is then applied to a lock of chestnut-brown pigmented natural Caucasian hair (TH4). The "mixture/lock" bath ratio is, respectively, 10/1 (g/g).

The leave-on time is 30 minutes on a hotplate set at 27°C, after which time the locks are rinsed and then washed with iNOA Post shampoo (L'Oréal Professionnel). Finally, the locks are dried under a hood at 40°C.

The colorimetric measurements were taken using a Datacolor Spectra Flash SF 600X colorimeter in the CIELab system (illuminant D65, angle 10°).

The lightening is represented by the difference in colour DE between the untreated locks of hair and the locks

of hair after treatment and is calculated according to the following equation:

$$DE^* = \sqrt{(L^* - L_o{}^*)^2 + (a^* - a_o{}^*)^2 + (b^* - b_o{}^*)^2} \quad (i)$$

In this equation, the parameters L*, a* and b* represent the values measured on locks after treatment and the parameters $L_0$*, $a_0$* and $b_0$* represent the values measured on the untreated locks. The higher the DE* value, the better the lightening of the keratin fibres.

Results:

| | L*( | a* | b*( | DE*( |
|---|---|---|---|---|
| Untreated pigmented natural hair (TH4) | 19.79 | 2.73 | 2.62 | |
| After treatment with the composition of the invention A1+B | 25.78 | 7.33 | 10.34 | **10.79** |
| After treatment with the comparative composition A2 + B | 24.63 | 6.92 | 8.53 | **8.71** |

The composition according to the invention derived from the mixing of compositions (A1) + B allows better lightening of the keratin fibres than the comparative composition derived from the mixing of compositions (A2) + (B).

## Example 2

The following compositions were prepared (amounts expressed in g unless otherwise mentioned):

| INCI names | B1 g% comparative | B2 g% invention |
|---|---|---|
| Guanidine Carbonate | 6.84 (0.076 mole) | 4 (0.044 mole) |
| Monoethanolamine | - | 2 (0.032 mole) |
| Ammonium hydroxide | 2.47 AM (0.07 mole) | 2.47 AM (0.07 mole) |
| Liquid petroleum jelly | 11,6 | 11,6 |
| Cetearyl alcohol | 6 | 6 |
| Camellia oil | 0,2 | 0,2 |
| HEXADIMETHRINE CHLORIDE (Mexomere PO from Chimex) | 0,3 MA | 0,3 MA |
| POLYQUATERNIUM-6 (Merquat 100 from Lubrizol) | 0.4 MA | 0.4 MA |
| STEARETH-2 | 1,38 | 1,38 |
| STEARETH-20 | 2,75 | 2,75 |
| Sodium metabisulfite | 0,7 | 0,7 |
| Erythorbic acid | 0,3 | 0,3 |
| Propylene glycol | 13 | 13 |
| EDTA | 0,2 | 0,2 |
| Fragrance | qs | qs |
| Water | Qsp 100 | Qsp 100 |

Oxidizing composition

| | g% |
|---|---|
| 50% Aqueous hydrogen peroxide solution | 6 AM |
| Tetrasodium pyrophosphate | 0.02 |
| Pentasodium pentetate | 0.15 |
| Glycerol | 0.5 |

(continued)

| | g% |
|---|---|
| Cetylstearyl alcohol/oxyethylenated (30 OE) cetylstearyl alcohol mixture (Nonidac 1618 F from Sasol) | 2.85 |
| Trideceth-2 carboxamide MEA (Amidet A15/LAO 55 from Kao) | 0.85 |
| Phosphoric acid | qs pH 2.2 |
| Sodium stannate | 0.04 |
| Water | qs 100 |

At the time of use, 1 part by weight of composition B1 or B2 is mixed with 1 part by weight of composition B. Each mixture is then applied to locks of chesnut natural hair (tone height of 4) at a rate of 10 g of mixture per 1 g of hair. The leave-on time is 30 minutes on a hotplate set at 27°C, after which time the locks are rinsed and then washed with conventional shampoo and dried .

The colorimetric measurements were taken using a Datacolor Spectra Flash SF 600X colorimeter in the CIELab system (illuminant D65, angle 10°).

The lightening is represented by the lightness L*

The following results are obtained :

| | L* |
|---|---|
| Non treated locks | 19.42 |
| B1+O mixture (comparative) | 24 |
| B2+O mixture (invention) | 26.26 |

The mixture B2+O according to the invention provides higher L* value, thus better lightening than the comparative mixture B1+O.

**Claims**

1. Composition, in particular for dyeing and/or lightening keratin fibres, in particular human keratin fibres such as the hair, comprising:

   a) one or more guanidine salts,
   b) one or more alkanolamine(s),
   c) ammonium hydroxide and
   d) at least two polymers chosen from cationic and amphoteric polymers, which are different from each other, the cationic polymers being chosen from

      (1) Homopolymers or copolymers derived from acrylic or methacrylic esters or amides and comprising at least one of the units of formula (I), (II), (III) or (IV) below:

(I) 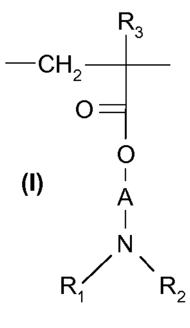

(II) 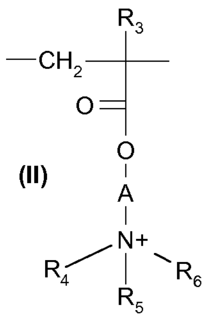

(III) 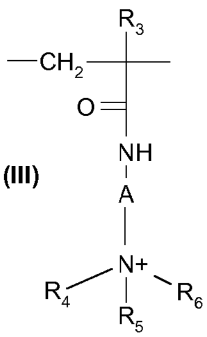

(IV) 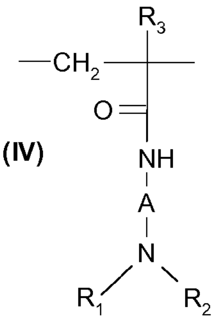

in which:

$R_3$, which may be identical or different, denote a hydrogen atom or a $CH_3$ radical;
A, which may be identical or different, represent a linear or branched $C_1$-$C_6$ and preferably $C_2$-$C_3$ alkyl group or a $C_1$-$C_4$ hydroxyalkyl group;
$R_4$, $R_5$ and $R_6$, which may be identical or different, represent a $C_1$-$C_{18}$ alkyl group or a benzyl radical, and preferably a $C_1$-$C_6$ alkyl group;
$R_1$ and $R_2$, which may be identical or different, represent hydrogen or a $C_1$-$C_6$ alkyl group, preferably methyl or ethyl;
X denotes an anion derived from a mineral or organic acid, such as a methosulfate anion or a halide such as chloride or bromide,

(2) Cationic cellulose derivatives such as cellulose copolymers or cellulose derivatives grafted with a water-soluble quaternary ammonium monomer,
(3) Cationic guar gums ,
(4) Polymers constituted of piperazinyl units and of divalent alkylene or hydroxyalkylene radicals containing straight or branched chains, optionally interrupted with oxygen, sulfur or nitrogen atoms or by aromatic or heterocyclic rings, and also the oxidation and/or quaternization products of these polymers,
(5) Water-soluble polyaminoamides ,
(6) Polymers obtained by reaction of a polyalkylene polyamine containing two primary amine groups and at least one secondary amine group with a dicarboxylic acid chosen from diglycolic acid and saturated $C_3$-$C_8$ aliphatic dicarboxylic acids. ,
(7) Cyclopolymers of alkyldiallylamine or of dialkyldiallylammonium, such as the homopolymers or copolymers containing, as main constituent of the chain, units corresponding to formula (V) or (VI):

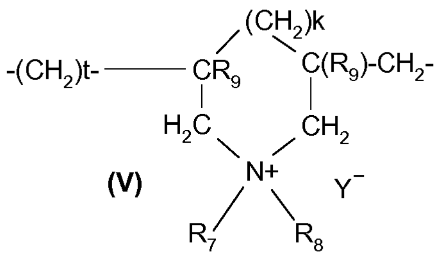
(V)

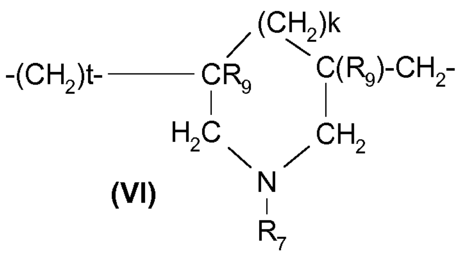
(VI)

in which formulae k and t are equal to 0 or 1, the sum k + t being equal to 1; $R_9$ denotes a hydrogen atom or a methyl radical; $R_7$ and $R_8$, independently of each other, denote a $C_1$-$C_8$ alkyl group, a hydroxyalkyl group in which the alkyl group is $C_1$-$C_5$, an amidoalkyl group in which the alkyl is $C_1$-$C_4$; $R_7$ and $R_8$ can also denote, together with the nitrogen atom to which they are attached, a heterocyclic group such as piperidyl or morpholinyl; $R_7$ and $R_8$, independently of each other, preferably denote a $C_1$-$C_4$ alkyl group; $Y^-$ is an organic or mineral anion such as bromide, chloride, acetate, borate, citrate, tartrate, bisulfate, bisulfite, sulfate or phosphate,
(8) The quaternary diammonium polymer containing repeating units corresponding to the formula:

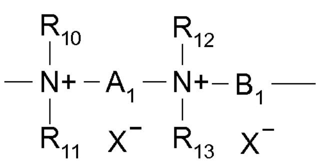
(VII)

in which formula:

$R_{10}$, $R_{11}$, $R_{12}$ and $R_{13}$, which may be identical or different, represent $C_1$-$C_{20}$ aliphatic, alicyclic or arylaliphatic radicals or hydroxyalkylaliphatic radicals in which the alkyl radical is $C_1$-$C_4$, or alternatively $R_{10}$, $R_{11}$, $R_{12}$ and $R_{13}$, together or separately, constitute, with the nitrogen atoms to which they are attached, heterocycles optionally containing a second heteroatom other than nitrogen, or alternatively $R_{10}$, $R_{11}$, $R_{12}$ and $R_{13}$ represent a linear or branched $C_1$-$C_6$ alkyl radical substituted with a nitrile, ester, acyl or amide group or a group -CO-O-$R_{14}$-D or -CO-NH-$R_{14}$-D where $R_{14}$ is an alkylene and D is a

quaternary ammonium group;
$A_1$ and $B_1$ represent linear or branched, saturated or unsaturated $C_2$-$C_{20}$ polymethylene groups which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and
$X^-$ denotes an anion derived from a mineral or organic acid;
$A_1$, $R_{10}$ and $R_{12}$ can form, with the two nitrogen atoms to which they are attached, a piperazine ring;
in addition, if $A_1$ denotes a linear or branched, saturated or unsaturated alkylene or hydroxyalkylene radical, $B_1$ may also denote a group $-(CH_2)_n$-CO-D-OC-$(CH_2)_n$- in which n is between 1 and 100 and preferably between 1 and 50, and D denotes:

a) a glycol residue of formula: -O-Z-O-, where Z denotes a linear or branched hydrocarbon-based radical or a group corresponding to one of the following formulae:
$-(CH_2-CH_2-O)_x-CH_2-CH_2-$ and $-[CH_2-CH(CH_3)-O]_y-CH_2-CH(CH_3)-$ where x and y denote an integer from 1 to 4, representing a defined and unique degree of polymerization or any number from 1 to 4 representing an average degree of polymerization;
b) a bis-secondary diamine residue, such as a piperazine derivative;
c) a bis-primary diamine residue of formula: -NH-Y-NH-, where Y denotes a linear or branched hydrocarbon-based radical, or alternatively the radical $-CH_2-CH_2-S-S-CH_2$
$-CH_2-$;
d) a ureylene group of formula: -NH-CO-NH-.

Preferably, $X^-$ is an anion, such as chloride or bromide,

(9) Polyquaternary ammonium polymers constituted of repeating units of formula (IX):

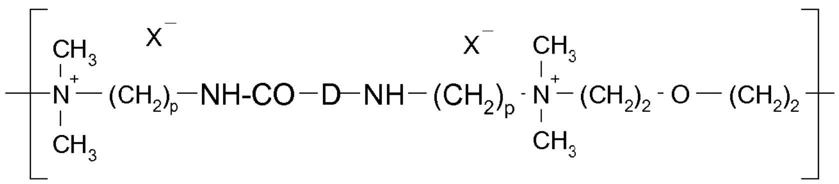

(IX)

in which p denotes an integer ranging from 1 to 6 approximately, D may be zero or may represent a group $-(CH_2)_r$-CO- in which r denotes a number equal to 4 or 7, and $X^-$ is an anion,
(10) Quaternary polymers of vinylpyrrolidone and of vinylimidazole,
(11) Polyamines,
and mixtures thereof,

e) optionally one or more colouring agents.

2. Composition according to Claim 1, **characterized in that** the guanidine salt(s) are chosen from inorganic guanidine salts, preferably from guanidine carbonate and guanidine hydrogen carbonate, better still guanidine carbonate.

3. Composition according to either of the preceding claims, **characterized in that** the guanidine salt(s) are present in a content ranging from 1.2% to 10% by weight and preferably from 1.5% to 7% by weight relative to the total weight of the composition.

4. Composition according to any one of the preceding claims, **characterized in that** the alkanolamine(s) are chosen from monoalkanolamines, dialkanolamines and trialkanolamines comprising from one to three identical or different $C_1$-$C_4$ hydroxyalkyl radicals.

5. Composition according to any one of the preceding claims, **characterized in that** the alkanolamine(s) are chosen from monoethanolamine, diethanolamine, triethanolamine, monoisopropanolamine, diisopropanolamine, N-dimethylaminoethanolamine, 2-amino-2-methyl-1-propanol, triisopropanolamine, 2-amino-2-methyl-1,3-propanediol, 3-amino-1,2-propanediol, 3-dimethylamino-1,2-propanediol and tris(hydroxymethyl)aminomethane; preferably the alkanolamine is monoethanolamine.

6. Composition according to any one of the preceding claims, **characterized in that** it has a total content of alkanolamine(s) ranging from 0.01% to 10% by weight, preferably from 0.1% to 7% by weight and better still from 0.5% to 5% by weight relative to the weight of said composition.

7. Composition according to any one of the preceding claims, **characterized in that** it has an ammonium hydroxide content of from 0.01% to 10% by weight, preferably from 0.1% to 10% by weight and more preferentially from 0.5% to 8% by weight relative to the total weight of the composition.

8. Composition according to any one of the preceding claims, **characterized in that** the cationic polymers are chosen from:

(7) cyclopolymers of alkyldiallylamine or of dialkyldiallylammonium, such as the homopolymers or copolymers containing, as main constituent of the chain, units corresponding to formula (V) or (VI):

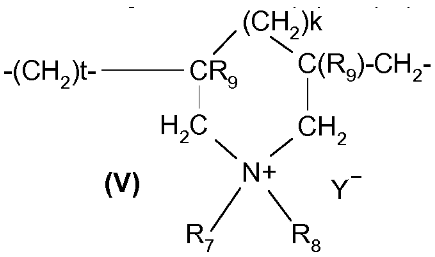
(V)

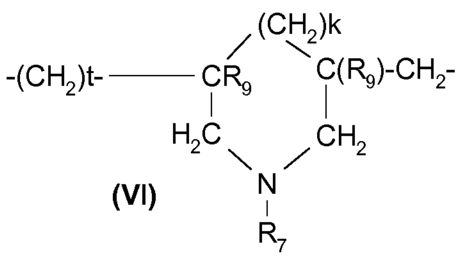
(VI)

in which formulae k and t are equal to 0 or 1, the sum k + t being equal to 1; $R_9$ denotes a hydrogen atom or a methyl radical; $R_7$ and $R_8$, independently of each other, denote a $C_1$-$C_8$ alkyl group, a hydroxyalkyl group in which the alkyl group is $C_1$-$C_5$, an amidoalkyl group in which the alkyl is $C_1$-$C_4$; $R_7$ and $R_8$ can also denote, together with the nitrogen atom to which they are attached, a heterocyclic group such as piperidyl or morpholinyl; $R_7$ and $R_8$, independently of each other, preferably denote a $C_1$-$C_4$ alkyl group; $Y^-$ is an organic or mineral anion such as bromide, chloride, acetate, borate, citrate, tartrate, bisulfate, bisulfite, sulfate or phosphate,
(8) quaternary diammonium polymers containing repeating units corresponding to the formula:

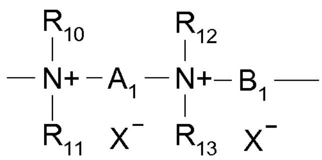
(VII)

in which formula:

$R_{10}$, $R_{11}$, $R_{12}$ and $R_{13}$, which may be identical or different, represent $C_1$-$C_{20}$ aliphatic, alicyclic or arylaliphatic radicals or hydroxyalkylaliphatic radicals in which the alkyl radical is $C_1$-$C_4$, or alternatively $R_{10}$, $R_{11}$, $R_{12}$ and $R_{13}$, together or separately, constitute, with the nitrogen atoms to which they are attached, heterocycles optionally containing a second heteroatom other than nitrogen, or alternatively $R_{10}$, $R_{11}$, $R_{12}$ and $R_{13}$ represent a linear or branched $C_1$-$C_6$ alkyl radical substituted with a nitrile, ester, acyl or amide group or a group -CO-O-$R_{14}$-D or -CO-NH-$R_{14}$-D where $R_{14}$ is an alkylene and D is a quaternary ammonium group;
$A_1$ and $B_1$ represent linear or branched, saturated or unsaturated $C_2$-$C_{20}$ polymethylene groups which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and
$X^-$ denotes an anion derived from a mineral or organic acid;
$A_1$, $R_{10}$ and $R_{12}$ can form, with the two nitrogen atoms to which they are attached, a piperazine ring;
in addition, if $A_1$ denotes a linear or branched, saturated or unsaturated alkylene or hydroxyalkylene radical, $B_1$ may also denote a group $-(CH_2)_n$-CO-D-OC-$(CH_2)_n$- in which n is between 1 and 100 and preferably between 1 and 50, and D denotes:

a) a glycol residue of formula: -O-Z-O-, where Z denotes a linear or branched hydrocarbon-based radical or a group corresponding to one of the following formulae:

$-(CH_2-CH_2-O)_x-CH_2-CH_2-$ and $-[CH_2-CH(CH_3)-O]_y-CH_2-CH(CH_3)-$ where x and y denote an integer from 1 to 4, representing a defined and unique degree of polymerization or any number from 1 to 4 representing an average degree of polymerization;
b) a bis-secondary diamine residue, such as a piperazine derivative;
c) a bis-primary diamine residue of formula: -NH-Y-NH-, where Y denotes a linear or branched hydrocarbon-based radical, or alternatively the radical $-CH_2-CH_2-S-S-CH_2-CH_2-$;
d) a ureylene group of formula: -NH-CO-NH-,
preferably, $X^-$ is an anion, such as chloride or bromide,
and, preferably, diquaternary ammonium polymers containing repeating units corresponding to formula (VIII) below:

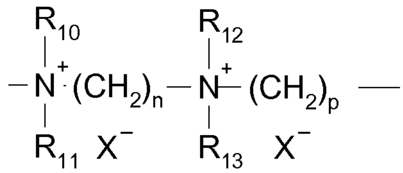

**(VIII)**

in which $R_{10}$, $R_{11}$, $R_{12}$ and $R_{13}$, which may be identical or different, denote a $C_1$-$C_4$ alkyl or hydroxyalkyl radical, n and p are integers ranging from 2 to 20 approximately, and $X^-$ is an anion derived from a mineral or organic acid.

(9) Polyquaternary ammonium polymers constituted of repeating units of formula (IX):

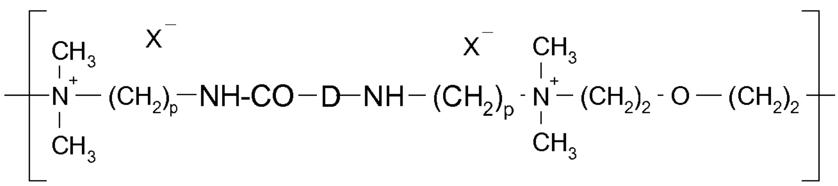

**(IX)**

in which p denotes an integer ranging from 1 to 6 approximately, D may be zero or may represent a group $-(CH_2)_r-CO-$ in which r denotes a number equal to 4 or 7, and $X^-$ is an anion.

9. Composition according to any one of the preceding claims, **characterized in that** the cationic polymers are chosen from the polymers of families (7) and (8) according to the preceding claim, alone or as mixtures, and even more preferentially from polymers bearing repeating units of formulae (W) and (U) below:

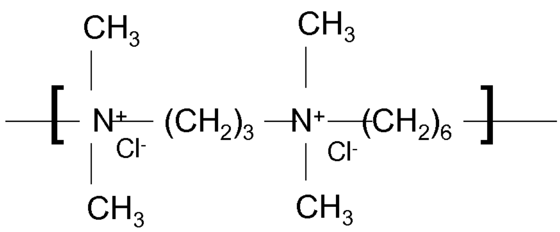

**(W)**

and in particular those of which the molecular weight, determined by gel permeation chromatography, is between 9500 and 9900;

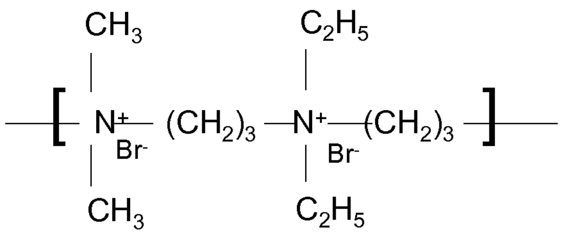

**(U)**

and especially those whose molecular weight, determined by gel permeation chromatography, is about 1200.

**10.** Composition according to any one of the preceding claims, **characterized in that** the amphoteric polymers are chosen from:

(1') polymers comprising as monomers at least one monomer derived from a vinyl compound carrying a carboxyl group, such as, more particularly, acrylic acid, methacrylic acid, maleic acid, alpha-chloroacrylic acid, and at least one basic monomer derived from a substituted vinyl compound containing at least one basic atom, chosen especially from the following:

a) dialkylaminoalkyl methacrylates, dialkylaminoalkyl acrylates, dialkylaminoalkylmethacrylamides and dialkylaminoalkylacrylamides,
b) trialkylaminoalkyl methacrylate salts and trialkylaminoalkyl acrylate salts, and salts of trialkylaminoalkylmethacrylamide and of trialkylaminoalkylacrylamide,

(3') copolymers comprising as monomers at least one monomer derived from a vinyl compound bearing a carboxylic group, such as, more particularly, acrylic acid, methacrylic acid, maleic acid, alpha-chloroacrylic acid, and at least one monomer of diallyldialkylammonium salt type, the alkyl groups containing from 1 to 6 carbon atoms, preferably, the alkyl group is a methyl group.

**11.** Composition according to any one of the preceding claims, **characterized in that** the amphoteric polymer(s) are chosen from acrylic acid/acrylamidopropyltrimethylammonium chloride copolymers, acrylic acid/acrylamidopropyltrimethylammonium chloride/acrylamide copolymers, copolymers comprising as monomers dimethyldiallylammonium chloride and acrylic acid optionally combined with acrylamide, and mixtures thereof, preferably the acrylic acid/acrylamidopropyltrimethylammonium chloride copolymer.

**12.** Composition according to any one of the preceding claims, **characterized in that** it comprises at least one cationic polymer and at least one amphoteric polymer, preferably chosen from the polymers defined in any one of Claims 8 to 11.

**13.** Composition according to any one of the preceding claims, **characterized in that** it comprises at least two cationic polymers which are different from each other, preferably chosen from the polymers defined in any one of Claims 8 to 11.

**14.** Composition according to any one of the preceding claims, **characterized in that** it comprises at least one cationic polymer chosen from the polymers of family (7) and at least one cationic polymer chosen from the polymers of family (8), in particular polymers bearing repeating units (W) or (U), preferably at least one cationic polymer chosen from dimethyldiallylammonium chloride homopolymers and diallyldimethylammonium chloride copolymers, and at least one polymer chosen from polymers bearing repeating units of formula (W), in particular the polymer whose INCI name is hexadimethrine chloride.

**15.** Composition according to any one of the preceding claims, **characterized in that** the total content of amphoteric and/or cationic polymers preferably represents from 0.01% to 15%, better still from 0.05% to 10% and even more preferentially from 0.1% to 5% by weight relative to the total weight of the composition.

**16.** Composition according to any one of the preceding claims, **characterized in that** the colouring agent is chosen from oxidation dye precursors and direct dyes, and mixtures thereof.

**17.** Composition according to any one of the preceding claims, **characterized in that** it comprises at least one oxidation dye chosen from oxidation bases and couplers, and mixtures thereof.

**18.** Composition according to any one of the preceding claims, **characterized in that** it comprises at least one saturated or unsaturated, linear or branched $C_2$-$C_8$ and preferably $C_3$-$C_6$ polyol, comprising from 2 to 6 hydroxyl groups.

**19.** Composition according to any one of the preceding claims, **characterized in that** the polyol(s) are chosen from glycerol, propylene glycol, 1,3-butylene glycol, dipropylene glycol and diglycerol, and mixtures thereof, and more preferably the polyol(s) are chosen from glycerol and propylene glycol, and mixtures thereof.

**20.** Composition according to any one of the preceding claims, **characterized in that** the total content of polyol(s) represents from 0.1% to 25% by weight, preferably from 1% to 20% by weight and more particularly from 2% to 15% by weight, relative to the weight of the composition.

**21.** Composition according to any one of the preceding claims, **characterized in that** it does not comprise any chemical oxidizing agent.

**22.** Composition according to any one of Claims 1 to 20, **characterized in that** it comprises a chemical oxidizing agent, preferably hydrogen peroxide optionally combined with one or more peroxygenated salts.

**23.** Process for dyeing and/or lightening keratin fibres, in particular human keratin fibres such as the hair, in which the composition as defined according to any one of Claims 1 to 20 is applied to said fibres, alone or in the presence of a chemical oxidizing agent.

**24.** Multi-compartment device comprising:
at least a first compartment containing:

- a composition (A) comprising:

  a) one or more guanidine salts as defined in any one of the preceding claims,
  b) one or more alkanolamines as defined in any one of the preceding claims,
  c) ammonium hydroxide and
  d) at least two polymers chosen from cationic and amphoteric polymers, which are different from each other, as defined in any one of the preceding claims,
  e) optionally one or more oxidation dye precursors and at least a second compartment containing a composition (B) comprising at least one chemical oxidizing agent.

**Patentansprüche**

**1.** Zusammensetzung, insbesondere zum Färben und/oder Aufhellen von Keratinfasern, insbesondere menschlichen Keratinfasern wie dem Haar, umfassend:

a) ein oder mehrere Guanidinsalze,
b) ein oder mehrere Alkanolamine,
c) Ammoniumhydroxid und
d) mindestens zwei Polymere, die aus kationischen und amphoteren Polymeren, die voneinander verschieden sind, ausgewählt sind, wobei die kationischen Polymere aus

(1) Homopolymeren oder Copolymeren, die sich von Acrylsäure- oder Methacrylsäureestern oder -amiden ableiten und mindestens eine der Einheiten der nachstehenden Formel (I), (II), (III) oder (IV) umfassen:

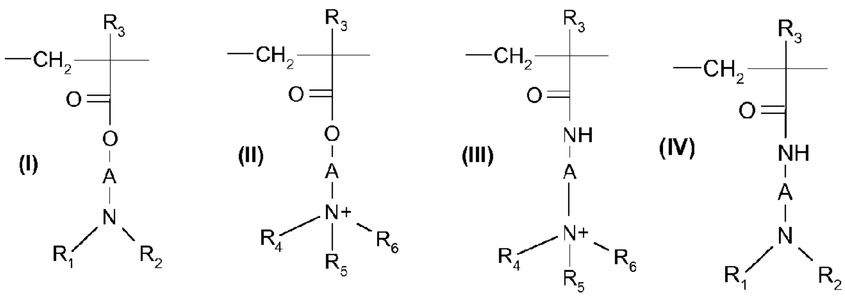

(I) (II) (III) (IV)

in denen:

$R_3$, das gleich oder verschieden sein kann, ein Wasserstoffatom oder einen $CH_3$-Rest bedeutet;
A, das in jeder Formel gleich oder verschieden sein kann, eine lineare oder verzweigte $C_1$-$C_6$- und vorzugsweise $C_2$-$C_3$-Alkylgruppe eine $C_1$-$C_4$-Hydroxyalkylgruppe bedeutet;

$R_4$, $R_5$ und $R_6$, die gleich oder verschieden sein können, für eine $C_1$-$C_{18}$-Alkylgruppe oder einen Benzylrest und vorzugsweise eine $C_1$-$C_6$-Alkylgruppe stehen;
$R_1$ und $R_2$, die gleich oder verschieden sein können, für ein Wasserstoffatom oder eine $C_1$-$C_6$-Alkylgruppe, vorzugsweise Methyl oder Ethyl, stehen;
X ein Anion, das sich von einer Mineralsäure oder organischen Säure ableitet, wie ein Methosulfat-Anion, oder ein Halogenid wie Chlorid oder Bromid bedeutet,

(2) kationischen Cellulosederivaten wie Cellulosecopolymeren oder Cellulosederivaten, die mit einem wasserlöslichen quaternären Ammonium-Monomer gepfropft sind,
(3) kationischen Guar-Gummen,
(4) Polymeren, die aus Piperazinyleinheiten und zweiwertigen Alkylen- oder Hydroxyalkylenresten mit geraden oder verzweigten Ketten, die gegebenenfalls durch Sauerstoff-, Schwefel- oder Stickstoffatome oder durch aromatische oder heterocyclische Ringe unterbrochen sind, bestehen, sowie den Oxidations- und/oder Quaternisierungsprodukten dieser Polymere,
(5) wasserlöslichen Polyaminoamiden,
(6) durch Reaktion eines Polyalkylenpolyamins mit zwei primären Aminogruppen und mindestens einer sekundären Amingruppe mit einer aus Diglykolsäure und gesättigten aliphatischen $C_3$-$C_8$-Dicarbonsäuren ausgewählten Dicarbonsäure erhaltenen Polymeren,
(7) Cyclopolymeren von Alkyldiallylamin oder Dialkyldiallylammonium, wie den Homopolymeren oder Copolymeren, die als Hauptbestandteil der Kette Einheiten umfassen, die der Formel (V) oder (VI) entsprechen:

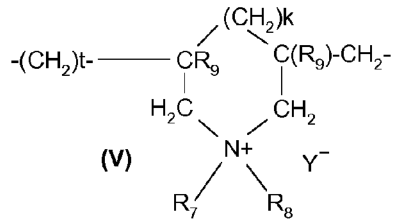
(V)

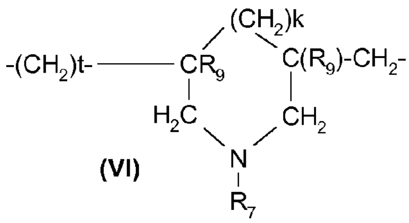
(VI)

in denen k und t gleich 0 oder 1 sind, die Summe k + t gleich 1 ist; $R_9$ für ein Wasserstoffatom oder einen Methylrest steht; $R_7$ und $R_8$ unabhängig voneinander eine $C_1$-$C_8$-Alkylgruppe, eine Hydroxyalkylgruppe mit 1 bis 5 Kohlenstoffatomen im Alkylteil oder eine Amidoalkylgruppe mit 1 bis 4 Kohlenstoffatomen im Alkylteil bedeuten; $R_7$ und $R_8$ auch zusammen mit dem Stickstoffatom, an das sie gebunden sind, eine heterocyclische Gruppe wie Piperidinyl oder Morpholinyl bedeuten können; $R_7$ und $R_8$ unabhängig voneinander vorzugsweise eine $C_1$-$C_4$-Alkylgruppe bedeuten; $Y^-$ für ein organisches oder mineralisches Anion wie Bromid, Chlorid, Acetat, Borat, Citrat, Tartrat, Hydrogensulfat, Hydrogensulfit, Sulfat oder Phosphat steht,
(8) dem quaternären Diammoniumpolymer mit Wiederholungseinheiten der Formel:

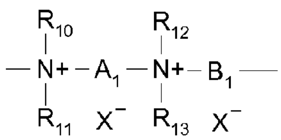
(VII)

in der:

$R_{10}$, $R_{11}$, $R_{12}$ und $R_{13}$, die gleich oder verschieden sein können, für aliphatische, alicyclische oder arylaliphatische $C_1$-$C_{20}$)-Reste oder hydroxyalkylaliphatische Reste mit 1 bis 4 Kohlenstoffatomen im Alkylteil stehen oder alternativ dazu $R_{10}$, $R_{11}$, $R_{12}$ und $R_{13}$ zusammen oder separat mit den Stickstoffatomen, an die sie gebunden sind, Heterocyclen bilden, die gegebenenfalls neben dem Stickstoff ein zweites Heteroatom enthalten, oder alternativ dazu $R_{10}$, $R_{11}$, $R_{12}$ und $R_{13}$ für einen linearen oder verzweigten $C_1$-$C_6$-Alkylrest stehen, der durch eine Nitril-, Ester-, Acyl- oder Amidgruppe oder eine Gruppe -CO-O-$R_{14}$-D oder -CO-NH-$R_{14}$-D substituiert ist, wobei $R_{14}$ für ein Alkylen steht und D für eine quaternäre Ammoniumgruppe steht;
$A_1$ und $B_1$ für lineare oder verzweigte, gesättigte oder ungesättigte $C_2$-$C_{20}$-Polymethylengruppen stehen, die an die Hauptkette gebunden oder in die Hauptkette eingeschoben einen oder mehrere aromatische Ringe oder ein oder mehrere Sauerstoff- oder Schwefelatome oder Sulfoxid-, Sulfon-, Disulfid-, Amino-, Alkylamino-, Hydroxyl-, quaternäre Ammonium-, Ureido-, Amid- oder Estergruppen enthalten können, und

$X^-$ ein Anion, das sich von einer Mineralsäure oder organischen Säure ableitet, bedeutet;

$A_1$, $R_{10}$ und $R_{12}$ mit den beiden Stickstoffatomen, die sie gebunden sind, einen Piperazinring bilden können;

außerdem dann, wenn $A_1$ einen linearen oder verzweigten, gesättigten oder ungesättigten Alkylen- oder Hydroxyalkylenrest bedeutet, $B_1$ auch eine Gruppe $-(CH_2)_n-CO-D-OC-(CH_2)_n-$ bedeuten kann, wobei n zwischen 1 und 100 liegt und D

a) einen Glykolrest der Formel: -O-Z-O-, wobei Z einen linearen oder verzweigten, gesättigten oder ungesättigten Rest auf Kohlenwasserstoffbasis oder eine Gruppe, die einer der folgenden Formeln entspricht, bedeutet:
$-(CH_2-CH_2-O)_x-CH_2-CH_2-$ und $-[CH_2-CH(CH_3)-O]_y-CH_2-CH(CH_3)-$, wobei x und y eine ganze Zahl von 1 bis 4, die einen definierten und einzigartigen Polymerisationsgrad wiedergeben, oder eine beliebige Zahl von 1 bis 4, die einen durchschnittlichen Polymerisationsgrad wiedergibt, bedeuten;
b) einen bissekundären Diaminrest, wie ein Piperazinderivat;
c) einen bisprimären Diaminrest der Formel: -NH-Y-NH-, wobei Y einen linearen oder verzweigten Rest auf Kohlenwasserstoffbasis oder alternativ dazu den Rest $-CH_2-CH_2-S-S-CH_2-CH_2-$ bedeutet;
d) eine Ureylengruppe der Formel: -NH-CO-NHbedeutet;

vorzugsweise $X^-$ ein Anion, wie Chlorid oder Bromid, bedeutet,

(9) polyquaternären Ammoniumpolymeren, die aus Wiederholungseinheiten der Formel (IX) bestehen:

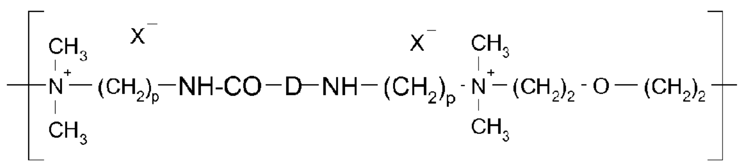

**(IX)**

worin p eine ganze Zahl im Bereich von ungefähr 1 bis 6 bedeutet, D null sein kann oder für eine Gruppe $-(CH_2)_r-CO-$, in der R eine Zahl mit einem Wert von 4 oder 7 bedeutet, stehen kann und $X^-$ für ein Anion steht,
(10) quaternären Polymeren von Vinylpyrrolidon und Vinylimidazol,
(11) Polyaminen
und Mischungen davon ausgewählt sind,

e) gegebenenfalls ein oder mehrere Färbemittel.

2. Zusammensetzung nach Anspruch 1, **dadurch gekennzeichnet, dass** das Guanidinsalz bzw. die Guanidinsalze aus anorganischen Guanidinsalzen, vorzugsweise aus Guanidincarbonat und Guanidinhydrogencarbonat, noch besser Guanidincarbonat, ausgewählt ist bzw. sind.

3. Zusammensetzung nach einem der vorhergehenden Ansprüche, **dadurch gekennzeichnet, dass** das Guanidinsalz bzw. die Guanidinsalze in einem Gehalt im Bereich von 1,2 bis 10 Gew.-% und vorzugsweise 1,5 bis 7 Gew.-%, bezogen auf das Gesamtgewicht der Zusammensetzung, vorliegt bzw. vorliegen.

4. Zusammensetzung nach einem der vorhergehenden Ansprüche, **dadurch gekennzeichnet, dass** das Alkanolamin bzw. die Alkanolamine aus Monoalkanolaminen, Dialkanolaminen und Trialkanolaminen mit einem bis drei gleichen oder verschiedenen $C_1$-$C_4$-Hydroxyalkylresten ausgewählt ist bzw. sind.

5. Zusammensetzung nach einem der vorhergehenden Ansprüche, **dadurch gekennzeichnet, dass** das Alkanolamin bzw. die Alkanolamine aus Monoethanolamin, Diethanolamin, Triethanolamin, Monoisopropanolamin, Diisopropanolamin, N-Dimethylaminoethanolamin, 2-Amino-2-methyl-1-propanol, Triisopropanolamin, 2-Amino-2-methyl-1,3-propandiol, 3-Amino-1,2-propandiol, 3-Dimethylamino-1,2-propandiol und Tris(hydroxymethyl)aminomethan ausgewählt ist bzw. sind; vorzugsweise das Alkanolamin Monoethanolamin ist.

**6.** Zusammensetzung nach einem der vorhergehenden Ansprüche, **dadurch gekennzeichnet, dass** sie einen Gesamtgehalt an Alkanolaminen im Bereich von 0,01 bis 10 Gew.-%, vorzugsweise von 0,1 bis 7 Gew.-% noch besser von 0,5 bis 5 Gew.-%, bezogen auf das Gewicht der Zusammensetzung, aufweist.

**7.** Zusammensetzung nach einem der vorhergehenden Ansprüche, **dadurch gekennzeichnet, dass** sie einen Ammoniumhydroxidgehalt von 0,01 bis 10 Gew.-%, vorzugsweise von 0,1 bis 10 Gew.-% und weiter bevorzugt von 0,5 bis 8 Gew.-%, bezogen auf das Gesamtgewicht der Zusammensetzung, aufweist.

**8.** Zusammensetzung nach einem der vorhergehenden Ansprüche, **dadurch gekennzeichnet, dass** die kationischen Polymere aus

(7) Cyclopoplymeren von Alkyldiallylamin oder Dialkyldiallylammonium, wie den Homopolymeren oder Copolymeren, die als Hauptbestandteil der Kette Einheiten umfassen, die der Formel (V) oder (VI) entsprechen:

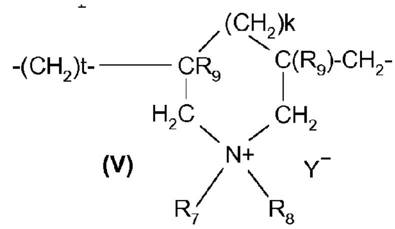
(V)

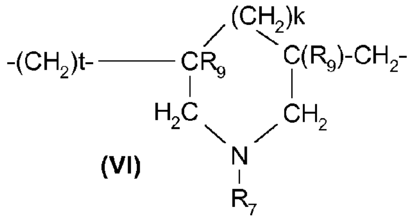
(VI)

in denen k und t gleich 0 oder 1 sind, die Summe k + t gleich 1 ist; $R_9$ für ein Wasserstoffatom oder einen Methylrest steht; $R_7$ und $R_8$ unabhängig voneinander eine $C_1$-$C_8$-Alkylgruppe, eine Hydroxyalkylgruppe mit 1 bis 5 Kohlenstoffatomen im Alkylteil oder eine Amidoalkylgruppe mit 1 bis 4 Kohlenstoffatomen im Alkylteil bedeuten; $R_7$ und $R_8$ auch zusammen mit dem Stickstoffatom, an das sie gebunden sind, eine heterocyclische Gruppe wie Piperidinyl oder Morpholinyl bedeuten können; $R_7$ und $R_8$ unabhängig voneinander vorzugsweise eine $C_1$-$C_4$-Alkylgruppe bedeuten; $Y^-$ für ein organisches oder mineralisches Anion wie Bromid, Chlorid, Acetat, Borat, Citrat, Tartrat, Hydrogensulfat, Hydrogensulfit, Sulfat oder Phosphat steht,
(8) quaternären Diammoniumpolymeren mit Wiederholungseinheiten der Formel:

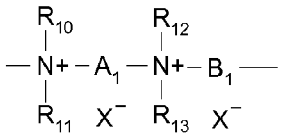
**(VII)**

in der:

$R_{10}$, $R_{11}$, $R_{12}$ und $R_{13}$, die gleich oder verschieden sein können, für aliphatische, alicyclische oder arylaliphatische $C_1$-$C_{20}$-Reste oder hydroxyalkylaliphatische Reste mit 1 bis 4 Kohlenstoffatomen im Alkylteil stehen oder alternativ dazu $R_{10}$, $R_{11}$, $R_{12}$ und $R_{13}$ zusammen oder separat mit den Stickstoffatomen, an die sie gebunden sind, Heterocyclen bilden, die gegebenenfalls neben dem Stickstoff ein zweites Heteroatom enthalten, oder alternativ dazu $R_{10}$, $R_{11}$, $R_{12}$ und $R_{13}$ für einen linearen oder verzweigten $C_1$-$C_6$-Alkylrest stehen, der durch eine Nitril-, Ester-, Acyl- oder Amidgruppe oder eine Gruppe -CO-O-$R_{14}$-D oder -CO-NH-$R_{14}$-D substituiert ist, wobei $R_{14}$ für ein Alkylen steht und D für eine quaternäre Ammoniumgruppe steht;
$A_1$ und $B_1$ für lineare oder verzweigte, gesättigte oder ungesättigte $C_2$-$C_{20}$-Polymethylengruppen stehen, die an die Hauptkette gebunden oder in die Hauptkette eingeschoben einen oder mehrere aromatische Ringe oder ein oder mehrere Sauerstoff- oder Schwefelatome oder Sulfoxid-, Sulfon-, Disulfid-, Amino-, Alkylamino-, Hydroxyl-, quaternäre Ammonium-, Ureido-, Amid- oder Estergruppen enthalten können, und $X^-$ ein Anion, das sich von einer Mineralsäure oder organischen Säure ableitet, bedeutet;
$A_1$, $R_{10}$ und $R_{12}$ mit den beiden Stickstoffatomen, an die sie gebunden sind, einen Piperazinring bilden können;
außerdem dann, wenn $A_1$ einen linearen oder verzweigten, gesättigten oder ungesättigten Alkylen- oder Hydroxyalkylenrest bedeutet, $B_1$ auch eine Gruppe - $(CH_2)_n$-CO-D-OC-$(CH_2)_n$- bedeuten kann, wobei n zwischen 1 und 100 liegt und vorzugsweise zwischen 1 und 50 liegt und D

a) einen Glykolrest der Formel: -O-Z-O-, wobei Z einen linearen oder verzweigten, gesättigten oder ungesättigten Rest auf Kohlenwasserstoffbasis oder eine Gruppe, die einer der folgenden Formeln entspricht, bedeutet:
$-(CH_2-CH_2-O)_x-CH_2-CH_2-$ und $-[CH_2-CH(CH_3)-O]_y-CH_2-CH(CH_3)-$, wobei x und y eine ganze Zahl von 1 bis 4, die einen definierten und einzigartigen Polymerisationsgrad wiedergeben, oder eine beliebige Zahl von 1 bis 4, die einen durchschnittlichen Polymerisationsgrad wiedergibt, bedeuten;
b) einen bissekundären Diaminrest, wie ein Piperazinderivat;
c) einen bisprimären Diaminrest der Formel: -NH-Y-NH-, wobei Y einen linearen oder verzweigten Rest auf Kohlenwasserstoffbasis oder alternativ dazu den Rest $-CH_2-CH_2-S-S-CH_2-CH_2-$ bedeutet;
d) eine Ureylengruppe der Formel: -NH-CO-NHbedeutet;

vorzugsweise $X^-$ ein Anion, wie Chlorid oder Bromid, bedeutet,
und vorzugsweise diquaternären Ammoniumpolymeren mit Wiederholungseinheiten der nachstehenden Formel (VIII):

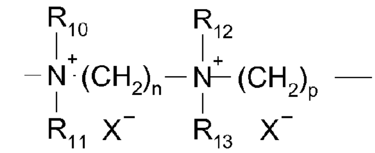
**(VIII)**

in der $R_{10}$, $R_{11}$, $R_{12}$ und $R_{13}$, die gleich oder verschieden sein können, einen $C_1$-$C_4$-Alkyl- oder -Hydroxyalkylrest bedeuten, n und p für ganze Zahlen im Bereich von ungefähr 2 bis 20 stehen und $X^-$ ein Anion, das sich von einer Mineralsäure oder organischen Säure ableitet, bedeutet,
(9) polyquaternären Ammoniumpolymeren, die aus Wiederholungseinheiten der Formel (IX) bestehen:

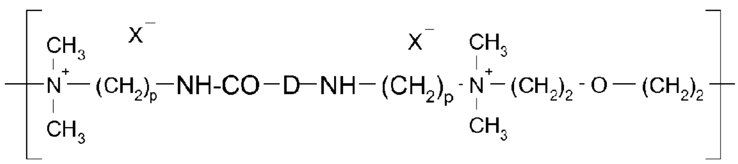
**(IX)**

worin p eine ganze Zahl im Bereich von ungefähr 1 bis 6 bedeutet, D null sein kann oder für eine Gruppe $-(CH_2)_r-CO-$, in der R eine Zahl mit einem Wert von 4 oder 7 bedeutet, stehen kann und $X^-$ für ein Anion steht, ausgewählt sind.

**9.** Zusammensetzung nach einem der vorhergehenden Ansprüche, **dadurch gekennzeichnet, dass** die kationischen Polymere aus den Polymeren der Familien (7) und (8) gemäß dem vorhergehenden Anspruch, alleine oder als Mischungen, und noch weiter bevorzugt aus Polymeren mit Wiederholungseinheiten der nachstehenden Formeln (W) und (U):

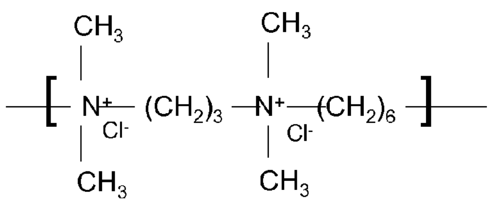
**(W)**

und insbesondere denjenigen, deren durch Gelpermeationschromatographie bestimmtes Molekulargewicht zwischen 9500 und 9900 liegt;

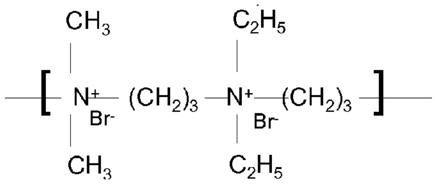

**(U)**

und insbesondere denjenigen, deren durch Gelpermeationschromatographie bestimmtes Molekulargewicht etwa 1200 beträgt,
ausgewählt sind.

10. Zusammensetzung nach einem der vorhergehenden Ansprüche, **dadurch gekennzeichnet, dass** die amphoteren Polymere aus

(1') Polymeren, die als Monomere mindestens ein Monomer, das sich von einer Vinylverbindung mit einer Carboxylgruppe, wie spezieller Acrylsäure, Methacrylsäure, Maleinsäure, alpha-Chloracrylsäure, ableitet, und mindestens ein basisches Monomer, das sich von einer substituierten Vinylverbindung mit mindestens einem basischen Atom ableitet und insbesondere aus den folgenden Monomeren ausgewählt ist:

a) Dialkylaminoalkylmethacrylaten, Dialkylaminoalkylacrylaten, Dialkylaminoalkylmethacrylamiden und Dialkylaminoalkylacrylamiden,
b) Trialkylaminoalkylmethacrylatsalzen und Trialkylaminoalkylacrylatsalzen und Salzen von Trialkylaminoalkylmethacrylamid und Trialkylaminoalkylacrylamid,
umfassen,

(3') Copolymeren, die als Monomere mindestens ein Monomer, das sich von einer Vinylverbindung mit einer Carboxylgruppe, wie spezieller Acrylsäure, Methacrylsäure, Maleinsäure, alpha-Chloracrylsäure, ableitet, und mindestens ein Monomer vom Diallyldialkylammoniumsalz-Typ, wobei die Alkylgruppen 1 bis 6 Kohlenstoffatome enthalten und vorzugsweise die Alkylgruppe eine Methylgruppe ist, umfassen,
ausgewählt sind.

11. Zusammensetzung nach einem der vorhergehenden Ansprüche, **dadurch gekennzeichnet, dass** das amphotere Polymer bzw. die amphoteren Polymere aus Acrylsäure/Acrylamidopropyltrimethylammoniumchlorid-Copolymeren, Acrylsäure/Acrylamidopropyltrimethylammoniumchlorid/Acrylamid-Copolymeren, Copolymeren, die als Monomere Dimethyldiallylammoniumchlorid und Acrylsäure, gegebenenfalls in Kombination mit Acrylamid, umfassen, und Mischungen davon, vorzugsweise dem Acrylsäure/Acrylamidopropyltrimethylammoniumchlorid-Copolymer, ausgewählt ist bzw. sind.

12. Zusammensetzung nach einem der vorhergehenden Ansprüche, **dadurch gekennzeichnet, dass** sie mindestens ein kationisches Polymer und mindestens ein amphoteres Polymer, die vorzugsweise aus den in einem der Ansprüche 8 bis 11 definierten Polymeren ausgewählt sind, umfasst.

13. Zusammensetzung nach einem der vorhergehenden Ansprüche, **dadurch gekennzeichnet, dass** es mindestens zwei kationische Polymere, die voneinander verschieden sind und vorzugsweise aus den Polymeren gemäß einem der Ansprüche 8 bis 11 ausgewählt sind, umfasst.

14. Zusammensetzung nach einem der vorhergehenden Ansprüche, **dadurch gekennzeichnet, dass** sie mindestens ein kationisches Polymer, das aus den Polymeren der Familie (7) ausgewählt ist, und mindestens ein kationisches Polymer, das aus den Polymeren der Familie (8), insbesondere Polymere mit Wiederholungseinheiten (W) oder (U), ausgewählt ist, vorzugsweise mindestens ein kationisches Polymer, das aus Dimethyldiallylammoniumchlorid-Homopolymeren und Diallyldimethylammoniumchlorid-Copolymeren ausgewählt ist, und mindestens ein Polymer, das aus Polymeren mit Wiederholungseinheiten der Formel (W), insbesondere dem Polymer, dessen INCI-Name Hexadimethrine Chloride lautet, umfasst.

15. Zusammensetzung nach einem der vorhergehenden Ansprüche, **dadurch gekennzeichnet, dass** der Gesamtgehalt an amphoteren und/oder kationischen Polymeren vorzugsweise im Bereich von 0,01 bis 15 Gew.-%, noch besser von 0,05 bis 10 Gew.-% und noch weiter bevorzugt von 0,1 bis 5 Gew.-%, bezogen auf das Gesamtgewicht der Zusammensetzung, liegt.

**16.** Zusammensetzung nach einem der vorhergehenden Ansprüche, **dadurch gekennzeichnet, dass** das Färbemittel aus Oxidationsfarbstoff-Vorstufen und Direktfarbstoffen und Mischungen davon ausgewählt ist.

**17.** Zusammensetzung nach einem der vorhergehenden Ansprüche, **dadurch gekennzeichnet, dass** sie mindestens einen Oxidationsfarbstoff umfasst, der aus Oxidationsbasen und Kupplern und Mischungen davon ausgewählt ist.

**18.** Zusammensetzung nach einem der vorhergehenden Ansprüche, **dadurch gekennzeichnet, dass** sie mindestens ein gesättigtes oder ungesättigtes, lineares oder verzweigtes $C_2$-$C_8$- und vorzugsweise $C_3$-$C_6$-Polyol mit 2 bis 6 Hydroxylgruppen umfasst.

**19.** Zusammensetzung nach einem der vorhergehenden Ansprüche, **dadurch gekennzeichnet, dass** das Polyol bzw. die Polyole aus Glycerin, Propylenglykol, 1,3-Butylenglykol, Dipropylenglykol und Diglycerin und Mischungen davon ausgewählt ist bzw. sind und weiter bevorzugt das Polyol bzw. die Polyole aus Glycerin und Propylenglykol und Mischungen davon ausgewählt ist bzw. sind.

**20.** Zusammensetzung nach einem der vorhergehenden Ansprüche, **dadurch gekennzeichnet, dass** der Gesamtgehalt an Polyol bzw. Polyolen 0,1 bis 25 Gew.-%, vorzugsweise 1 bis 20 Gew.-% und spezieller 2 bis 15 Gew.-%, bezogen auf das Gewicht der Zusammensetzung, ausmacht.

**21.** Zusammensetzung nach einem der vorhergehenden Ansprüche, **dadurch gekennzeichnet, dass** sie kein chemisches Oxidationsmittel umfasst.

**22.** Zusammensetzung nach einem der Ansprüche 1 bis 20, **dadurch gekennzeichnet, dass** sie ein chemisches Oxidationsmittel, vorzugsweise Wasserstoffperoxid, gegebenenfalls in Kombination mit einem oder mehreren peroxygenierten Salzen, umfasst.

**23.** Verfahren zum Färben und/oder Aufhellen von Keratinfasern, insbesondere menschlichen Keratinfasern wie dem Haar, bei dem man die Zusammensetzung gemäß einem der Ansprüche 1 bis 20 alleine oder in Gegenwart eines chemischen Oxidationsmittels auf die Fasern aufbringt.

**24.** Vorrichtung mit mehreren Kompartimenten, umfassend:

mindestens ein erstes Kompartiment, enthaltend:

- eine Zusammensetzung (A), enthaltend:

a) ein oder mehrere Guanidinsalze gemäß einem der vorhergehenden Ansprüche,
b) ein oder mehrere Alkanolamine gemäß einem der vorhergehenden Ansprüche,
c) Ammoniumhydroxid und
d) mindestens zwei Polymere, die aus kationischen und amphoteren Polymeren, die voneinander verschieden sind, ausgewählt sind, gemäß einem der vorhergehenden Ansprüche,
e) gegebenenfalls eine oder mehrere Oxidationsfarbstoff-Vorstufen und

mindestens ein zweites Kompartiment, enthaltend eine Zusammensetzung (B), die mindestens ein chemisches Oxidationsmittel umfasst.

## Revendications

**1.** Composition, en particulier pour la coloration et/ou l'éclaircissement de fibres kératiniques, en particulier de fibres kératiniques humaines telles que les cheveux, comprenant :

a) un ou plusieurs sels de guanidine,
b) une ou plusieurs alcanolamine(s),
c) de l'hydroxyde d'ammonium et
d) au moins deux polymères choisis parmi des polymères cationiques et amphotères, qui sont différents l'un de l'autre, les polymères cationiques étant choisis parmi

(1) des homopolymères ou copolymères issus d'esters ou d'amides acryliques ou méthacryliques et comportant au moins l'un des motifs de formule (I), (II), (III) ou (IV) ci-dessous :

(I)
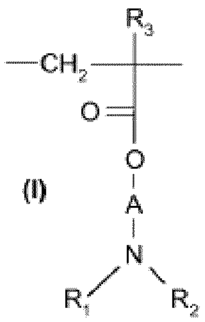

(II)
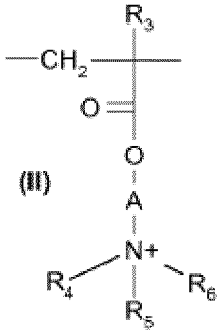

(III)
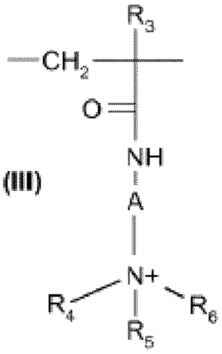

(IV)
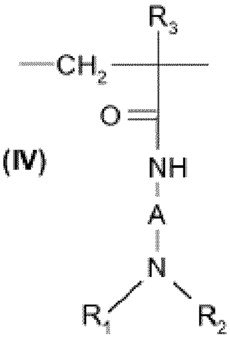

dans lesquelles:

$R_3$, qui peut être identique ou différent, désigne un atome d'hydrogène ou un radical $CH_3$ ;
A, qui peut être identique ou différent, représente un groupe alkyle, linéaire ou ramifié, en $C_1$-$C_6$, préférablement en $C_2$-$C_3$ ou un groupe $C_1$-$C_4$ hydroxyalkyle ;
$R_4$, $R_5$, et $R_6$, qui peuvent être identiques ou différents, représentent un groupe $C_1$-$C_{18}$ alkyle ou un radical benzyle, et de préférence un groupe $C_1$-$C_6$ alkyle ;
$R_1$ et $R_2$, qui peuvent être identiques ou différents, représentent hydrogène ou un groupe $C_1$-$C_6$ alkyle, préférablement méthyle ou éthyle;
X désigne un anion issu d'un acide minéral ou organique tel qu'un anion méthosulfate ou un halogénure tel que chlorure ou bromure,

(2) des dérivés de cellulose cationiques tels que des copolymères de cellulose ou des dérivés de cellulose greffés avec un monomère de type ammonium quaternaire soluble dans l'eau,
(3) des gommes de guar cationiques,
(4) des polymères constitués de motifs pipérazinyle et de radicaux alkylène ou hydroxyalkylène divalents contenant des chaînes droites ou ramifiées, éventuellement interrompues par des atomes d'oxygène, de soufre ou d'azote ou par des cycles aromatiques ou hétérocycliques, et également des produits d'oxydation et/ou de quaternisation de ces polymères,
(5) des polyaminoamides solubles dans l'eau,
(6) des polymères obtenus par réaction d'une polyalkylène polyamine contenant deux groupes amine primaire et au moins un groupe amine secondaire avec un acide dicarboxylique choisi parmi l'acide diglycolique et des acides dicarboxyliques aliphatiques saturés en $C_3$-$C_8$ ;
(7) des cyclopolymères d'alkyldiallylamine ou de dialkyldiallylammonium, tels que des homopolymères ou copolymères contenant, en tant que constituant principal de la chaîne, des motifs correspondant à la formule (V) ou (VI) :

(V)
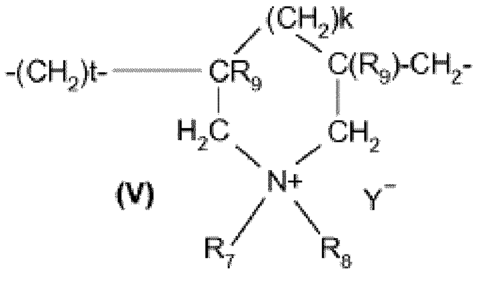

(VI)
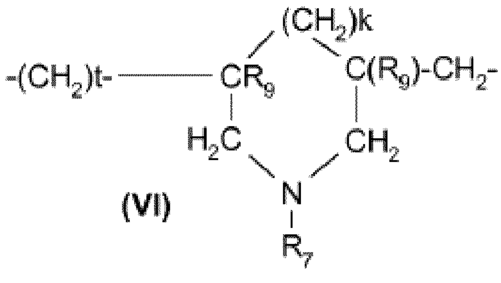

dans lesquelles k et t sont égaux à 0 ou 1, la somme k + t étant égale à 1 ; Rg désigne un atome d'hydrogène ou un radical méthyle ; $R_7$ et $R_8$, indépendamment l'un de l'autre, désignent un groupe $C_1$-$C_8$ alkyle, un groupe hydroxyalkyle dans lequel le groupe alkyle est $C_1$-$C_5$, un groupe amidoalkyle dans lequel l'alkyle est $C_1$-$C_4$ ; $R_7$ et $R_8$ peuvent également désigner, conjointement avec l'atome d'azote auquel ils sont fixés, un groupe hétérocyclique tel que pipéridinyle ou morpholinyle ; $R_7$ et $R_8$, indépendamment l'un de l'autre,

désignent préférablement un groupe $C_1$-$C_4$ alkyle ; $Y^-$ est un anion organique ou minéral tel que bromure, chlorure, acétate, borate, citrate, tartrate, bisulfate, bisulfite, sulfate ou phosphate,

(8) le polymère de type diammonium quaternaire contenant des motifs répétitifs correspondant à la formule :

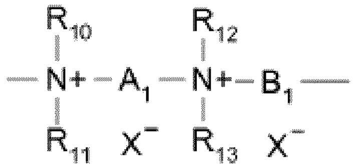

(VII)

dans laquelle :

$R_{10}$, $R_{11}$, $R_{12}$ et $R_{13}$, qui peuvent être identiques ou différents, représentent des radicaux aliphatiques, alicycliques ou arylaliphatiques en $C_1$-$C_{20}$ ou des radicaux hydroxyalkylaliphatiques dans lesquels le radical alkyle est $C_1$-$C_4$, ou en variante, $R_{10}$, $R_{11}$, $R_{12}$ et $R_{13}$, conjointement ou séparément, constituent, avec les atomes d'azote auxquels ils sont fixés, des hétérocycles contenant éventuellement un deuxième hétéroatome différent de l'azote, ou en variante, $R_{10}$, $R_{11}$, $R_{12}$ et $R_{13}$ représentent un radical $C_1$-$C_6$ alkyle linéaire ou ramifié substitué par un groupe nitrile, ester, acyle ou amide ou un groupe -CO-O-$R_{14}$-D ou -CO-NH-$R_{14}$-D dans lesquels $R_{14}$ est un alkylène et D est un groupe ammonium quaternaire ;
$A_1$ et $B_1$ représentent des groupes $C_2$-$C_{20}$ polyméthylène linéaires ou ramifiés, saturés ou insaturés, qui peuvent contenir, liés à la chaîne principale ou intercalés dans la chaîne principale, un ou plusieurs cycles aromatiques ou un ou plusieurs atomes d'oxygène ou de soufre ou groupes sulfoxyde, sulfone, disulfure, amino, alkylamino, hydroxyle, ammonium quaternaire, uréido, amide ou ester, et
$X^-$ désigne un anion issu d'un acide minéral ou organique ;
$A_1$, $R_{10}$ et $R_{12}$ peuvent former, avec les deux atomes d'azote auxquels ils sont fixés, un cycle pipérazine ; de plus, si $A_1$ désigne un radical alkylène ou hydroxyalkylène linéaire ou ramifié, saturé ou insaturé, $B_1$ peut également désigner un groupe $-(CH_2)_n$-CO-D-OC-$(CH_2)_n$- dans lequel n est compris entre 1 et 100 et préférablement entre 1 et 50, et D désigne :

a) un radical glycol de formule : -O-Z-O, dans laquelle Z désigne un radical à base d'hydrocarbure linéaire ou ramifié ou un groupe correspondant à l'une des formules suivantes :
$-(CH_2-CH_2-O)_x-CH_2-CH_2-$ ; - $[CH_2-CH(CH_3)-O]_y-CH_2-CH(CH_3)-$ dans lesquelles x et y désignent un entier de 1 à 4, représentant un degré de polymérisation défini et unique ou un quelconque nombre de 1 à 4 représentant un degré moyen de polymérisation ;
b) un radical de type diamine bis-secondaire, tel qu'un dérivé de pipérazine ;
c) un radical de type diamine bis-primaire de formule : -NH-Y-NH-, dans laquelle Y désigne un radical à base d'hydrocarbure linéaire ou ramifié, ou en variante le radical-$CH_2-CH_2-S-S-CH_2-CH_2-$ ;
d) un groupe uréylène de formule : -NH-CO-NH-, préférablement, $X^-$ est un anion, tel que chlorure ou bromure,

(9) des polymères de polyammonium quaternaire constitués de motifs répétitifs de formule (IX) :

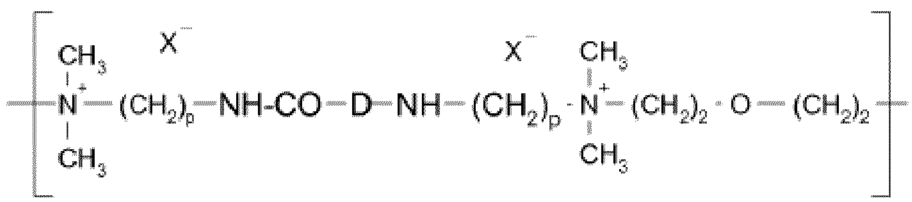

(IX)

dans laquelle p désigne un entier dans la plage de 1 à 6 approximativement, D peut être zéro ou peut représenter un groupe $-(CH_2)_r$-CO- dans lequel r désigne un nombre égal à 4 ou 7, et $X^-$ est un anion,
(10) des polymères quaternaires de vinylpyrrolidone et de vinylimidazole,
(11) des polyamines,
et des mélanges correspondants,

e) éventuellement un ou plusieurs agents de coloration.

**2.** Composition selon la revendication 1, **caractérisée en ce que** le ou les sels de guanidine sont choisis parmi des sels de guanidine inorganiques, préférablement parmi le carbonate de guanidine et l'hydrogénocarbonate de guanidine, encore mieux le carbonate de guanidine.

**3.** Composition selon l'une ou l'autre des revendications précédentes, **caractérisée en ce que** le ou les sels de guanidine sont présents en une teneur dans la plage de 1,2 % à 10 % en poids et préférablement de 1,5 % à 7 % en poids par rapport au poids total de la composition.

**4.** Composition selon l'une quelconque des revendications précédentes, **caractérisée en ce que** l'alcanolamine ou les alcanolamines sont choisies parmi des monoalcanolamines, des dialcanolamines et des trialcanolamines comprenant d'un à trois radicaux $C_1$-$C_4$ hydroxyalkyle identiques ou différents.

**5.** Composition selon l'une quelconque des revendications précédentes, **caractérisée en ce que** l'alcanolamine ou les alcanolamines sont choisies parmi la monoéthanolamine, la diéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol et le tris-hydroxyméthylaminométhane ; préférablement l'alcanolamine est la monoéthanolamine.

**6.** Composition selon l'une quelconque des revendications précédentes, **caractérisée en ce qu'**elle possède une teneur totale en alcanolamine(s) dans la plage de 0,01 % à 10 % en poids, préférablement de 0,1 % à 7 % en poids et mieux encore de 0,5 % à 5% en poids par rapport au poids de ladite composition.

**7.** Composition selon l'une quelconque des revendications précédentes, **caractérisée en ce qu'**elle possède une teneur en hydroxyde d'ammonium allant de 0,01 % à 10 % en poids, préférablement de 0,1 % à 10 % en poids et plus préférentiellement de 0,5 % à 8 % en poids, par rapport au poids total de la composition.

**8.** Composition selon l'une quelconque des revendications précédentes, **caractérisée en ce que** les polymères cationiques sont choisis parmi :

(7) des cyclopolymères d'alkyldiallylamine ou de dialkyldiallylammonium, tels que des homopolymères ou copolymères contenant, en tant que constituant principal de la chaîne, des motifs correspondant à la formule (V) ou (VI) :

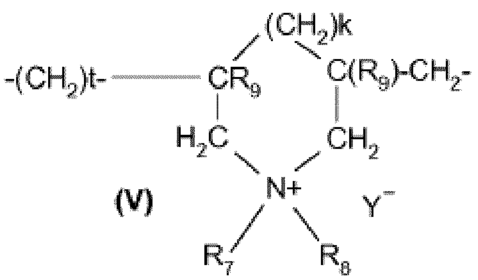
(V)

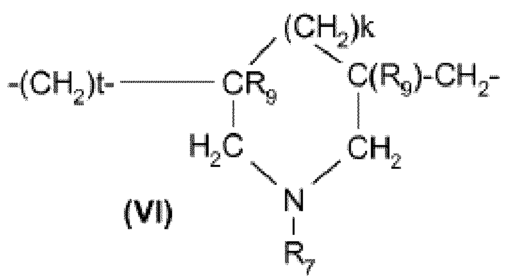
(VI)

dans lesquelles k et t sont égaux à 0 ou 1, la somme k + t étant égale à 1 ; Rg désigne un atome d'hydrogène ou un radical méthyle ; $R_7$ et $R_8$, indépendamment l'un de l'autre, désignent un groupe $C_1$-$C_8$ alkyle, un groupe hydroxyalkyle dans lequel le groupe alkyle est $C_1$-$C_5$, un groupe amidoalkyle dans lequel l'alkyle est $C_1$-$C_4$ ; $R_7$ et $R_8$ peuvent également désigner, conjointement avec l'atome d'azote auquel ils sont fixés, un groupe hétérocyclique tel que pipéridinyle ou morpholinyle ; $R_7$ et $R_8$, indépendamment l'un de l'autre, désignent préférablement un groupe $C_1$-$C_4$ alkyle ; $Y^-$ est un anion organique ou minéral tel que bromure, chlorure, acétate, borate, citrate, tartrate, bisulfate, bisulfite, sulfate ou phosphate,

(8) des polymères de type diammonium quaternaire contenant des motifs correspondant à la formule :

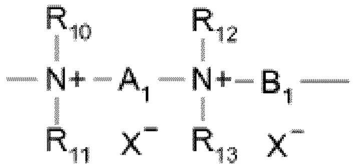

**(VII)**

dans laquelle :

$R_{10}$, $R_{11}$, $R_{12}$ et $R_{13}$, qui peuvent être identiques ou différents, représentent des radicaux aliphatiques, alicycliques ou arylaliphatiques en $C_1$-$C_{20}$ ou des radicaux hydroxyalkylaliphatiques dans lesquels le radical alkyle est $C_1$-$C_4$, ou en variante, $R_{10}$, $R_{11}$, $R_{12}$ et $R_{13}$, conjointement ou séparément, constituent, avec les atomes d'azote auxquels ils sont fixés, des hétérocycles contenant éventuellement un deuxième hétéroatome différent de l'azote, ou en variante, $R_{10}$, $R_{11}$, $R_{12}$ et $R_{13}$ représentent un radical $C_1$-$C_6$ alkyle linéaire ou ramifié substitué par un groupe nitrile, ester, acyle ou amide ou un groupe -CO-O-$R_{14}$-D ou -CO-NH-$R_{14}$-D dans lesquels $R_{14}$ est un alkylène et D est un groupe ammonium quaternaire ;
$A_1$ et $B_1$ représentent des groupes $C_2$-$C_{20}$ polyméthylène linéaires ou ramifiés, saturés ou insaturés qui peuvent contenir, liés à la chaîne principale ou intercalés dans la chaîne principale, un ou plusieurs cycles aromatiques ou un ou plusieurs atomes d'oxygène ou de soufre ou groupes sulfoxyde, sulfone, disulfure, amino, alkylamino, hydroxyle, ammonium quaternaire, uréido, amide ou ester, et
$X^-$ désigne un anion issu d'un acide minéral ou organique ;
$A_1$, $R_{10}$ et $R_{12}$ peuvent former, avec les deux atomes d'azote auxquels ils sont fixés, un cycle pipérazine ; de plus, si $A_1$ désigne un radical alkylène ou hydroxyalkylène linéaire ou ramifié, saturé ou insaturé, $B_1$ peut également désigner un groupe-$(CH_2)_n$-CO-D-OC-$(CH_2)_n$- dans lequel n est compris entre 1 et 100 et préférablement entre 1 et 50, et D désigne :

a) un radical glycol de formule : -O-Z-O, dans laquelle Z désigne un radical à base d'hydrocarbure linéaire ou ramifié ou un groupe correspondant à l'une des formules suivantes :
$-(CH_2-CH_2-O)_x-CH_2-CH_2-$ ; - $[CH_2-CH(CH_3)-O]_y-CH_2-CH(CH_3)-$ dans lesquelles x et y désignent un entier de 1 à 4, représentant un degré de polymérisation défini et unique ou un quelconque nombre de 1 à 4 représentant un degré moyen de polymérisation ;
b) un radical de type diamine bis-secondaire, tel qu'un dérivé de pipérazine ;
c) un radical de type diamine bis-primaire de formule : -NH-Y-NH-, dans laquelle Y désigne un radical à base d'hydrocarbure linéaire ou ramifié, ou en variante le radical $-CH_2-CH_2-S-S-CH_2-CH_2-$ ;
d) un groupe uréylène de formule : -NH-CO-NH-, préférablement, $X^-$ est un anion, tel que chlorure ou bromure,

et, préférablement des polymères d'ammonium diquaternaire contenant des motifs répétitifs correspondant à la formule (VIII) ci-dessous :

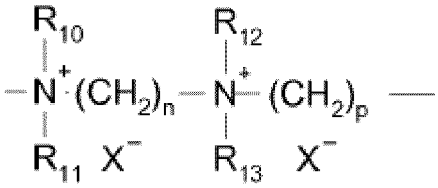

**(VIII)**

dans laquelle $R_{10}$, $R_{11}$, $R_{12}$ et $R_{13}$, qui peuvent être identiques ou différents, désignent un radical $C_1$-$C_4$ alkyle ou $C_1$-$C_4$ hydroxyalkyle, n et p sont des entiers dans la plage de 2 à 20 approximativement, et $X^-$ est un anion issu d'un acide minéral ou organique,
(9) des polymères de polyammonium quaternaire constitués de motifs répétitifs de formule (IX) :

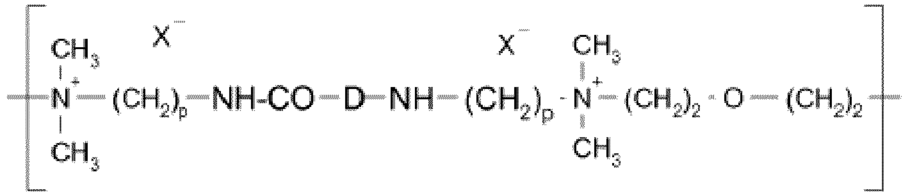

**(IX)**

dans laquelle p désigne un entier dans la plage de 1 à 6 approximativement, D peut être zéro ou peut représenter un groupe $-(CH_2)_r-CO-$ dans lequel r désigne un nombre égal à 4 ou 7, et $X^-$ est un anion.

**9.** Composition selon l'une quelconque des revendications précédentes, **caractérisée en ce que** les polymères cationiques sont choisis parmi les polymères des familles (7) et (8) selon la revendication précédente, seuls ou en tant que mélanges, et encore plus préférentiellement parmi des polymères portant des motifs répétitifs de formules (W) et (U) ci-dessous :

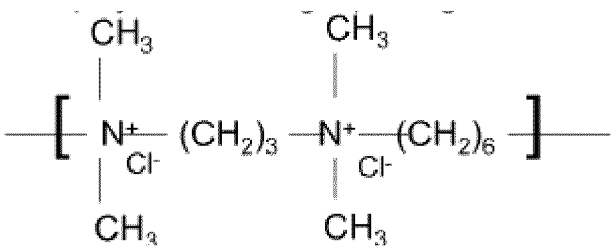

**(W)**

et en particulier ceux dont le poids moléculaire, déterminé par chromatographie à perméation de gel, est compris entre 9 500 et 9 900 ;

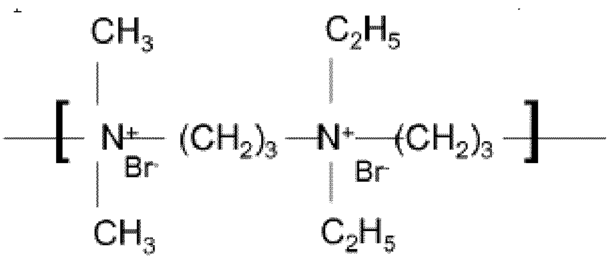

**(U)**

et notamment ceux dont le poids moléculaire, déterminé par chromatographie à perméation de gel, est d'environ 1 200.

**10.** Composition selon l'une quelconque des revendications précédentes, **caractérisée en ce que** les polymères amphotères sont choisis parmi :

(1') des polymères comprenant en tant que monomères au moins un monomère issu d'un composé vinylique portant un groupe carboxylique, tel que, plus particulièrement, l'acide acrylique, l'acide méthacrylique, l'acide maléique, l'acide alpha-chloracrylique, et au moins un monomère basique issu d'un composé vinylique substitué contenant au moins un atome basique, choisi notamment parmi :

a) des méthacrylates de dialkylaminoalkyle, des acrylates de dialkylaminoalkyle, des dialkylaminoalkylméthacrylamides et des dialkylaminoalkylacrylamides,
b) des sels de méthacrylate de trialkylaminoalkyle et des sels d'acrylate de trialkylaminoalkyle, et des sels de trialkylaminoalkylméthacrylamide et de trialkylaminoalkylacrylamide,

(3') des copolymères comprenant en tant que monomères au moins un monomère issu d'un composé vinylique portant un groupe carboxylique, tel que, plus particulièrement, l'acide acrylique, l'acide méthacrylique, l'acide maléique, l'acide alpha-chloracrylique, et au moins un monomère de type sel de diallyldialkylammonium, les groupes alkyle contenant de 1 à 6 atomes de carbone, préférablement, le groupe alkyle étant un groupe méthyle.

**11.** Composition selon l'une quelconque des revendications précédentes, **caractérisée en ce que** le ou les polymères amphotères sont choisis parmi des copolymères d'acide acrylique/chlorure d'acrylamidopropyltriméthylammonium, des copolymères d'acide acrylique/chlorure d'acrylamidopropyltriméthylammonium/acrylamide, des copolymères comprenant en tant que monomères du chlorure de diméthyldiallylammonium et de l'acide acrylique éventuellement combinée à de l'acrylamide, et des mélanges correspondants, préférablement le copolymère d'acide acrylique/chlorure d'acrylamidopropyltriméthylammonium.

**12.** Composition selon l'une quelconque des revendications précédentes, **caractérisée en ce qu'**elle comprend au moins un polymère cationique et au moins un polymère amphotère, préférablement choisis parmi les polymères définis dans l'une quelconque des revendications 8 à 11.

**13.** Composition selon l'une quelconque des revendications précédentes, **caractérisée en ce qu'**elle comprend au moins deux polymères cationiques qui sont différents l'un de l'autre, préférablement choisis parmi les polymères définis dans l'une quelconque des revendications 8 à 11.

**14.** Composition selon l'une quelconque des revendications précédentes, **caractérisée en ce qu'**elle comprend au moins un polymère cationique choisi parmi les polymères de la famille (7) et au moins un polymère cationique choisi parmi les polymères de la famille (8), en particulier des polymères portant des motifs répétitifs (W) ou (U), préférablement au moins un polymère cationique choisi parmi des homopolymères de chlorure de diméthyldiallylammonium et des copolymères de chlorure de diallyldiméthylammonium, et au moins un polymère choisi parmi des polymères portant des motifs répétitifs de formule (W), en particulier le polymère dont la dénomination INCI est hexadimethrine chloride.

**15.** Composition selon l'une quelconque des revendications précédentes, **caractérisée en ce que** la teneur totale en polymères amphotères et/ou cationiques représente préférablement de 0,01 % à 15 %, encore mieux de 0,05 % à 10 % et encore plus préférentiellement de 0,1 % à 5 % en poids, par rapport au poids total de la composition.

**16.** Composition selon l'une quelconque des revendications précédentes, **caractérisée en ce que** l'agent de coloration est choisi parmi des précurseurs de colorant d'oxydation et des colorants directs, et des mélanges correspondants.

**17.** Composition selon l'une quelconque des revendications précédentes, **caractérisée en ce qu'**elle comprend au moins un colorant d'oxydation choisi parmi des bases d'oxydation et des coupleurs, et des mélanges correspondants.

**18.** Composition selon l'une quelconque des revendications précédentes, **caractérisée en ce qu'**elle comprend au moins un polyol en $C_2$-$C_8$, et préférablement en $C_3$-$C_6$, saturé ou insaturé, linéaire ou ramifié, comprenant de 2 à 6 groupes hydroxyle.

**19.** Composition selon l'une quelconque des revendications précédentes, **caractérisée en ce que** le ou les polyols sont choisis parmi le glycérol, le propylène glycol, le 1,3-butylène glycol, le dipropylène glycol, le diglycérol, et des mélanges correspondants, et plus préférablement le ou les polyols sont choisis parmi le glycérol et le propylène glycol, et des mélanges correspondants.

**20.** Composition selon l'une quelconque des revendications précédentes, **caractérisée en ce que** la teneur totale en polyol(s) représente de 0,1 % à 25 % en poids, préférablement de 1 % à 20% en poids et plus particulièrement de 2 % à 15% en poids, par rapport au poids de la composition.

**21.** Composition selon l'une quelconque des revendications précédentes, **caractérisée en ce qu'**elle ne comprend aucun agent oxydant chimique.

**22.** Composition selon l'une quelconque des revendications 1 à 20, **caractérisée en ce qu'**elle comprend un agent oxydant chimique, préférablement le peroxyde d'hydrogène éventuellement combiné avec un ou plusieurs sels peroxygénés.

**23.** Procédé pour la coloration et/ou l'éclaircissement de fibres kératiniques, en particulier de fibres kératiniques humaines telles que les cheveux, dans lequel la composition telle que définie selon l'une quelconque des revendications 1 à 20 est appliquée auxdites fibres, seule ou en présence d'un agent oxydant chimique.

**24.** Dispositif à plusieurs compartiments comprenant :

au moins un premier compartiment contenant :

- une composition (A) comprenant :

  a) un ou plusieurs sels de guanidine tels que définis dans l'une quelconque des revendications précédentes,
  b) une ou plusieurs alcanolamines telles que définies dans l'une quelconque des revendications précédentes,
  c) de l'hydroxyde d'ammonium et
  d) au moins deux polymères choisis parmi des polymères cationiques et amphotères, qui sont différents l'un de l'autre, tels que définis dans l'une quelconque des revendications précédentes,
  e) éventuellement un ou plusieurs précurseurs de colorant d'oxydation, et
  au moins un deuxième compartiment contenant une composition (B) comprenant au moins un agent oxydant chimique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 3015240 A1
- EP 80976 A
- FR 2077143
- FR 2393573
- US 4131576 A
- US 3589578 A
- US 4031307 A
- FR 2162025
- FR 2280361
- FR 2252840
- FR 2368508
- FR 1583363
- US 3227615 A
- US 2961347 A
- FR 2080759
- FR 2190406
- FR 2320330
- FR 2270846
- FR 2316271
- FR 2336434
- FR 2413907
- US 2273780 A
- US 2375853 A
- US 2388614 A
- US 2454547 A
- US 3206462 A
- US 2261002 A
- US 2271378 A
- US 3874870 A
- US 4001432 A
- US 3929990 A
- US 3966904 A
- US 4005193 A
- US 4025617 A
- US 4025627 A
- US 4025653 A
- US 4026945 A
- US 4027020 A
- US 4157388 A
- US 4702906 A
- US 4719282 A
- EP 122324 A
- US 3836537 A
- FR 1400366
- GB 1026978 A
- GB 1153196 A
- FR 2801308
- DE 2359399
- JP 63169571 A
- JP 5063124 A
- EP 0770375 A
- WO 9615765 A
- US 4874554 A
- US 4137180 A


### Non-patent literature cited in the description


- **WALTER.** Noll's Chemistry and Technology of Silicones. Academic Press, 1968
- Volatile Silicone Fluids for Cosmetics. Cosmetics and Toiletries. Todd & Byers, January 1976, vol. 91, 27-32
- **M.R. PORTER.** Handbook of Surfactants. Blackie & Son, 1991, 116-178